# EUROPEAN PATENT APPLICATION

(11) **EP 4 220 174 A1**
(43) Date of publication of application: **02.08.2023**
(21) Application number: 23154569.0
(22) Date of filing: 01.02.2023
(51) Int. Cl.: G01N 33/68, H01J 49/00

(54) **ANTIPHOSPHOLIPID ANTIBODIES FOR THE DIAGNOSIS OF LYME DISEASE**

(30) Priority: 01.02.2022 US 202263267412 P
(71) Applicant: Trustees of Tufts College, Medford, MA 02155 (US)
(72) Inventor: Gwynne, Peter, Boston, 02111 (US); Hu, Linden, Boston, 02111 (US)
(74) Representative: Longland, Emma Louise

(57) **Abstract**

Disclosed herein are compositions and methods for diagnosing Lyme disease, including diagnosis within the first two weeks of infection. Also disclosed herein are compositions and methods for detecting and diagnosing infection by a *Borrelia sp.*

## Description

### CROSS-REFERENCE TO RELATED APPLCIATIONS

This application claims the benefit of U.S. Provisional Application No. 63/267,412 filed February 1, 2022, the content of which is incorporated herein by reference in its entirety.

### STATEMENT REGARDING FEDERALLY SPONSORED RESEARCH OR DEVELOPMENT

This invention was made with government support under AI150157, AI109656 and AI122286 awarded by the National Institute of Health. The government has certain rights in the invention.

### BACKGROUND

Lyme disease is the most common vector-borne disease in the United States. Lyme disease is caused by the bacterium *Borrelia burgdorferi* and less often by *Borrelia mayonii.* In Europe, the disease is typically caused by *B. afzelii* or *B. garinii.* It is transmitted to humans through the bite of infected blacklegged ticks. Typical symptoms include fever, headache, fatigue, and a characteristic skin rash called erythema migrans. If left untreated, infection can spread to joints, the heart, and the nervous system. Lyme disease is diagnosed based on symptoms, physical findings (e.g., rash), and the possibility of exposure to infected ticks. There is a need in the art for additional methods of detecting Lyme disease and *Borrelia* infections, including, e.g., for earlier diagnosis, increased detection sensitivity, tracking responses to therapies, and diagnosis of reinfection.

### SUMMARY

Disclosed herein are compositions and methods for the detection of Lyme disease in a subject.

In a first aspect, provided herein is a method of measuring the level of one or more antiphospholipid antibodies in a sample, the method comprising contacting the sample with a selected lipid, incubating to allow the lipid to be bound by antibodies, if present, and measuring the level of antibodies bound to the lipid using molecular detection. The level of antibodies bound to the lipid can then be compared to a predetermined value to inform a diagnosis or treatment decision, such as, a diagnosis of Lyme disease or a *Borrelia* infection.

In one aspect, provided herein is a method comprising (a) contacting an antibody-containing sample, derived from a subject, with a lipid panel configured on a solid support, the lipid panel comprising lipids: phosphatidic acid (PA), phosphatidyl choline (PC), and phosphatidylserine (PS), and optionally one or more of ceramide, sphingomyelin, hexosyl cholesterol, acyl hexosyl cholesterol, galactosyldiacylglycerol, or phosphatidylethanolamine; (b) incubating the sample with the lipids to allow binding of antibodies in the sample to the lipids; (c) contacting the bound antibodies with a detectable binding agent; (d) detecting the detectable binding agent; (e) measuring the level of antibodies bound to each of the lipids based on the detecting step (d); (f) comparing the measured levels of step (e) to a predetermined value. In some embodiments, the sample comprises a blood, plasma, or serum sample. In some embodiments, the solid support comprises a bead, a plate, or a flow cell. In some embodiments, the subject is human and the detectable binding agent comprises anti-human IgG comprising a detectable label (e.g., IgG conjugated to a detectable agent). In some embodiments, the detectable agent comprises an enzyme, such as a horseradish peroxidase. In some embodiments, if the measured level is greater than the predetermined value for at least one of the lipids in the panel (e.g., PA, PC, and/or PS), the subject is diagnosed as positive for Lyme disease. In some embodiments, if the measured level is less than the predetermined value for each of the lipids in the panel, the subject is diagnosed as negative for Lyme disease. In some embodiments, if the measured level is greater than the predetermined value for at least one of the lipids in the panel, the subject is diagnosed as positive for Lyme disease and the method further comprises treating the subject for Lyme disease.

In some aspects, provided herein is a method comprising (a) contacting an antibody-containing sample, derived from a subject, with a reagent composition comprising the following lipids: phosphatidic acid (PA), phosphatidyl choline (PC), and phosphatidylserine (PS), and optionally containing one or more additional lipids selected from ceramide, sphingomyelin, hexosyl cholesterol, acyl hexosyl cholesterol, galactosyldiacylglycerol, or phosphatidylethanolamine; (b) incubating the sample with the reagent composition to allow binding of antibodies in the sample, if present, to the lipids thereby forming an antibody-lipid complex; (c) detecting bound antibodies (e.g., antibody-lipid complexes); (d) measuring the level of antibodies bound to PA, PC, and PS, and the optional one or more additional lipids based on the detecting step (c); and (e) comparing the measured levels of (d) to a predetermined value for PA, PC, and PS, and the optional one or more additional lipids. In some embodiments, the detecting step (c) comprises detecting the bound antibodies (e.g., the antibody-lipid complexes) with a detectable binding agent. In some embodiments, the detecting step (c) comprises flowing the reagent-composition through a flow-chamber. In some embodiments, the detection comprises a microfluidic chamber, microchip comprising a plurality of microfluidic chambers, or lateral flow assay. In some embodiments, the method comprises, after the incubating step (b) and before the detecting step (c), the step of washing away unbound antibodies. In some embodiments, if the measured level is greater than the predetermined value for at least one of the lipids in the panel, the subject is diagnosed as positive for Lyme disease. In some embodiments, if the measured level is less than the predetermined value for each of the lipids in the panel, the subject is diagnosed as negative for Lyme disease. In some embodiments, if the measured level is greater than the predetermined value for at least one of the lipids in the panel, the subject is diagnosed as positive for Lyme disease and the method further comprises treating the subject for Lyme disease.

In another aspect, provided herein is a method comprising (a) contacting an antibody-containing sample, derived from a subject, with a lipid panel configured on a solid support, the lipid panel comprising lipids: phosphatidic acid (PA), phosphatidyl choline (PC), and phosphatidylserine (PS), and optionally one or more additional lipids selected from ceramide, sphingomyelin, hexosyl cholesterol, acyl hexosyl cholesterol, galactosyldiacylglycerol, or phosphatidylethanolamine; (b) incubating the sample with the lipids to allow binding of antibodies in the sample to the lipids; (c) contacting the bound antibodies with a detectable binding agent; (d) detecting the detectable binding agent; (e) measuring the level of antibodies bound to each of the lipids based on the detecting step (d); (f) comparing the measured levels of step (e) to a previously measured level of anti- PA, PC, and/or PS antibodies in the subject, and/or to a previously measured level of antibodies bound to the one or more additional lipids. In some embodiments, the subject is being treated for Lyme disease and if the measured level is less than a previously measured level for at least one of the lipids in the panel, the subject is responding positively to a therapy and/or is clearing an infection by a *Borrelia sp.* In some embodiments, if the measured level is statistically less by at least 5%, 10%, 15%, 20%, or more than a previously measured value for at least one of the lipids in the panel, the subject is responding positively to a therapy and/or is clearing an infection by a *Borrelia sp.*

In another aspect, provided herein is a composition, system, or kit comprising one or more lipids. In some embodiments, the composition, system or kit comprises a lipid panel. In some embodiments, the lipid panel is configured on a solid support. In some embodiments, th the lipid panel comprises phosphatidic acid (PA), phosphatidyl choline (PC), and phosphatidylserine (PS), and optionally one or more additional lipids selected from ceramide, sphingomyelin, hexosyl cholesterol, acyl hexosyl cholesterol, galactosyldiacylglycerol, or phosphatidylethanolamine. In some embodiments, the solid support comprises a bead, plate, or flow cell.

In some embodiments, a composition, system, or kit provided herein comprises: phosphatidic acid (PA), phosphatidyl choline (PC), phosphatidylserine (PS); and at least one of a synthetic buffer, stabilizer, non-specific binding surface blocking agent, macromolecular crowding agent, antioxidant, preservative, and surfactant. In some embodiments, the PA, PC, and PS are immobilized on a solid support. In some embodiments, the solid support comprises a plate, bead, flow chamber, microfluidic chamber, or microchip comprising a plurality of microfluidic chambers. In some embodiments, the composition, kit or system additionally comprises one or more additional lipids selected from ceramide, sphingomyelin, hexosyl cholesterol, acyl hexosyl cholesterol, galactosyldiacylglycerol, or phosphatidylethanolamine.

Provided herein is a lyophilized composition comprising the lipids: phosphatidic acid (PA), phosphatidyl choline (PC), and phosphatidylserine (PS). In some embodiments, the lyophilized composition further comprises a buffer, stabilizer, non-specific binding surface blocking agent, macromolecular crowding agent, antioxidant, preservative, and/or surfactant. In some embodiments, the composition additionally comprises one or more additional lipids selected from ceramide, sphingomyelin, hexosyl cholesterol, acyl hexosyl cholesterol, galactosyldiacylglycerol, or phosphatidylethanolamine.

In another aspect, provided herein is composition, system or kit comprising a lipid composition described herein and one additional reagent or device, such as a flow chamber, beads, ELISA reagent, anti-human IgG antibody, and/or HRP substrate. In some embodiments, a composition, system or kit is provided, comprising instructions for using the included components in the performance of a method described herein. In some embodiments, the compositions, systems, or kits disclosed herein comprise positive and/or negative control samples.

### BRIEF DESCRIPTION OF THE FIGURES

The detailed description is described with reference to the accompanying figures. The use of the same reference numbers in different instances in the description and the figures may indicate similar or identical items. Various embodiments or examples ("examples") of the present disclosure are disclosed in the following detailed description and the accompanying drawings. The drawings are not necessarily to scale. In general, operations of disclosed processes may be performed in an arbitrary order, unless otherwise provided in the claims.

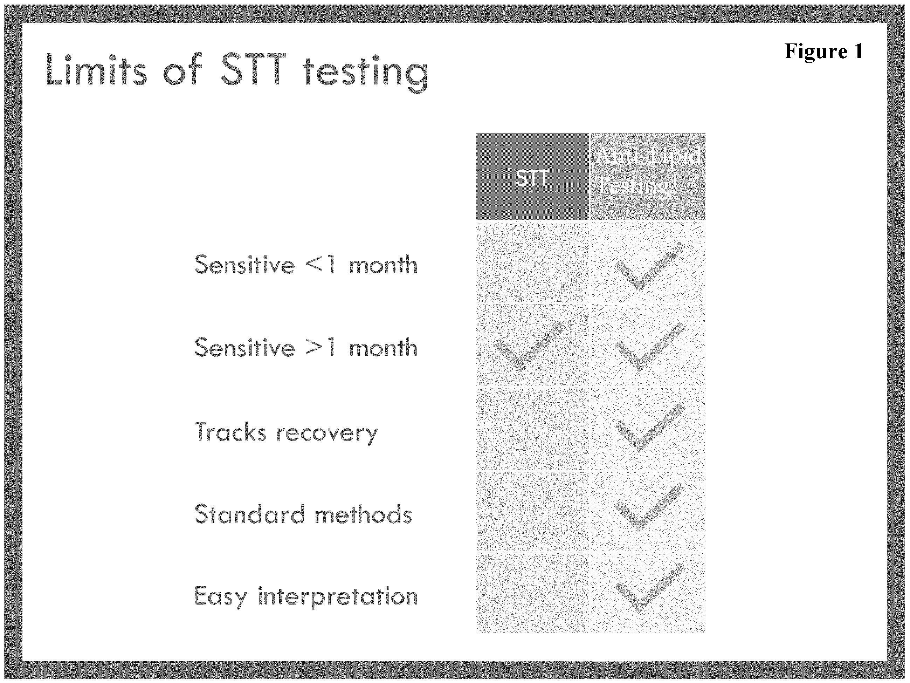

**Figure 1** is a drawing illustrating the limits of the current testing methods for Lyme diseases, the standard two-tier test (STT). SST testing is good at one month but has some major limitations. The methods disclosed herein, some of which are referred to as the aP test, use different antibody biomarkers, improves on several performance characteristics, provides a simpler test for clinical labs and physicians, and offers the possibility of at-home testing.

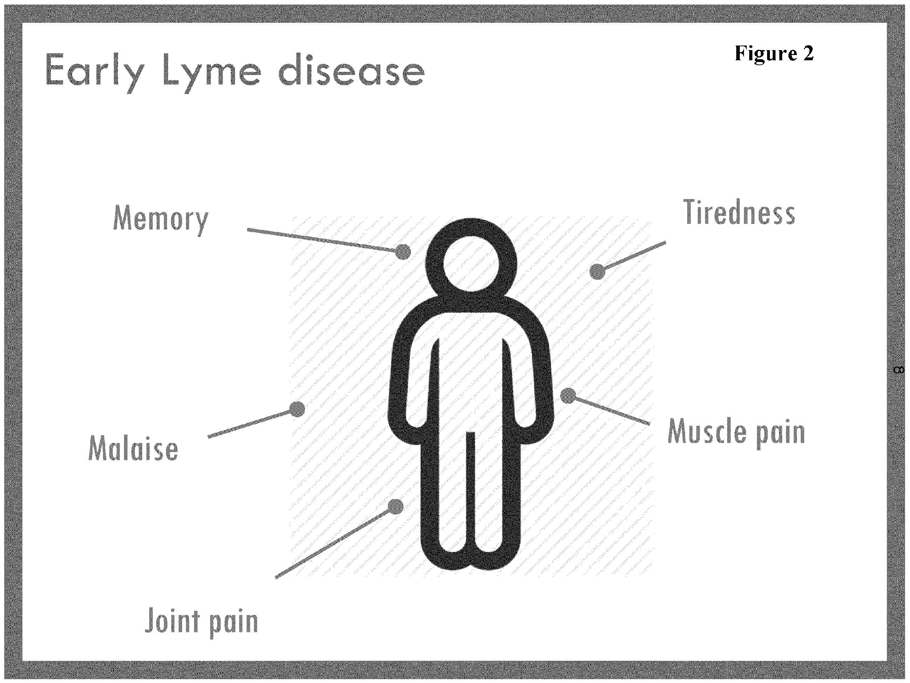

**Figure 2** is a drawing illustrating that early Lyme disease presents with very vague, generic symptoms. Particularly for non-specialists or those outside Lyme hot spots, it is not easy to make the right diagnosis clinically.

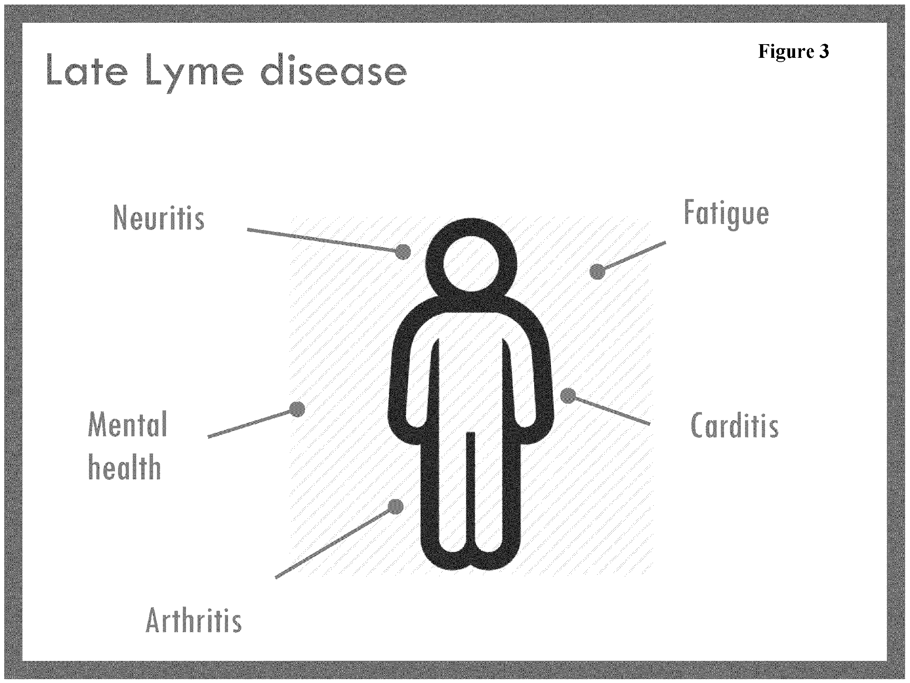

**Figure 3** is a drawing illustrating that later symptoms in Lyme disease often becomes more serious, and may include arrhythmia, arthritis, partial paralysis.

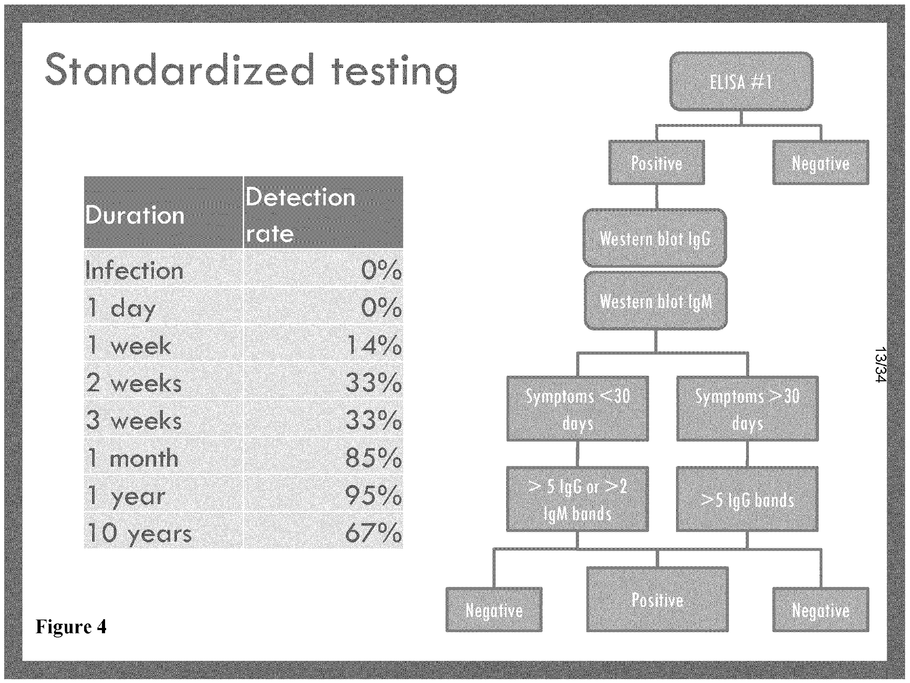

**Figure 4**. Is a drawing illustrating that complex testing algorithm presents a challenge - Western blots are not routinely used for any other tests. Western blots are incredibly difficult to interpret and often result in incorrect diagnoses. If a test is negative, health care providers will routinely wait a week and test again. Some will treat prospectively for Lyme, others will not - mostly based on guesswork. Numbers from Branda and Steere, 2021, and references therein (Branda, J., Steere, A., Clin Microbiol Rev 34(2): e00018-19 (2021)).

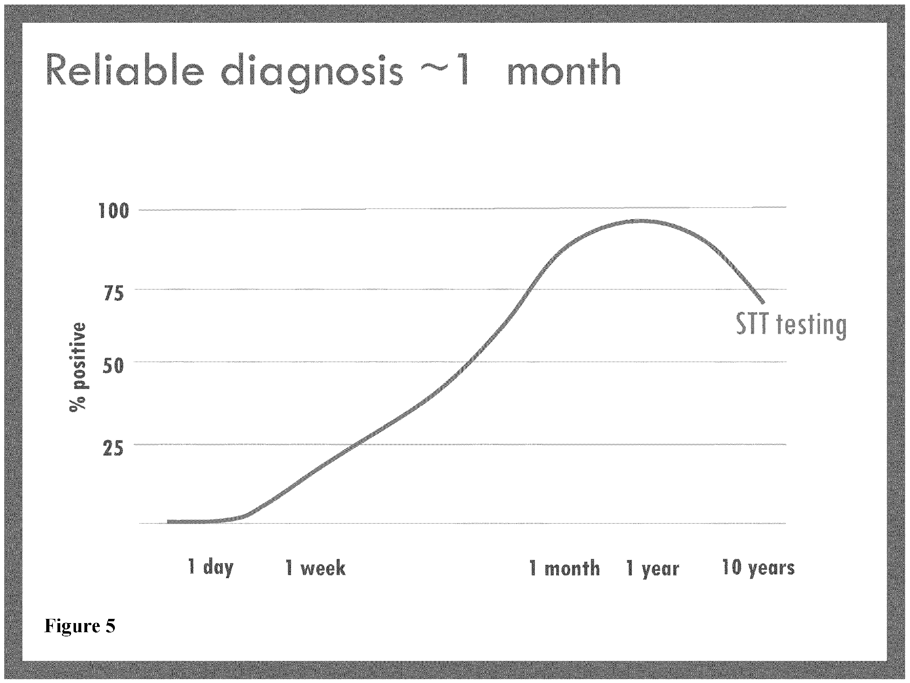

**Figure 5** is a graph showing the two areas of weakness with the SST testing: early and late. The fact that you remain positive after treatment causes two problems: there is no metric for clearance of the infection. This can result in repeated long courses of antibiotics, despite no evidence of either persistent infection or any benefit to patients. Re-testing someone who has had an infection in the past is virtually useless - current tests cannot distinguish a new infection from one that happened 20 years ago. This is a huge problem, for example, for a gardener from Maine who may be exposed every year.

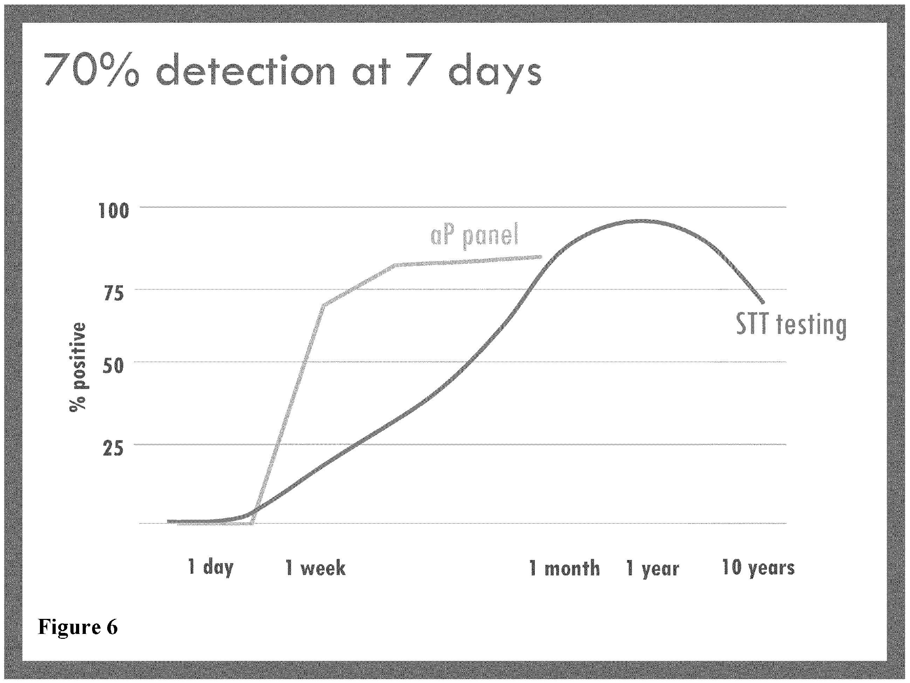

**Figure 6** is a graph illustrating that the methods of the present technology (termed the aP test, or aP panel) is more sensitive and faster. STT testing is 14% at 7 days, aP testing gets 70% at the same point. By 1 month the aP test about the same 83% vs STT 85%.

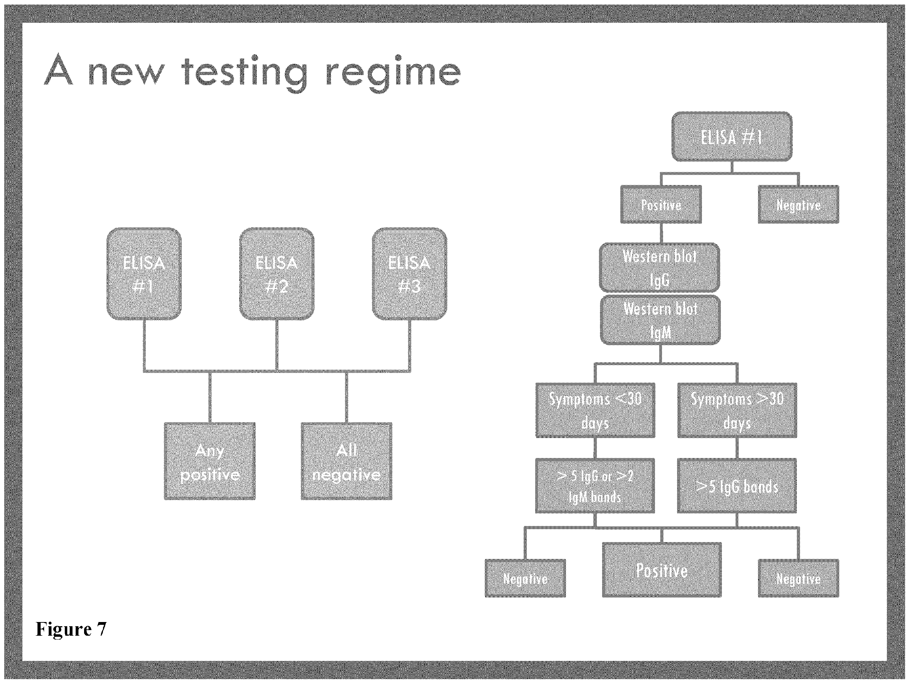

**Figure 7** is a schematic illustrating the aP testing algorithm (left side) is much simpler than the SST algorithm. At its most complex, clinicians would get three "yes" or "no" boxes rather than spotting/counting bands on a Western blot.8

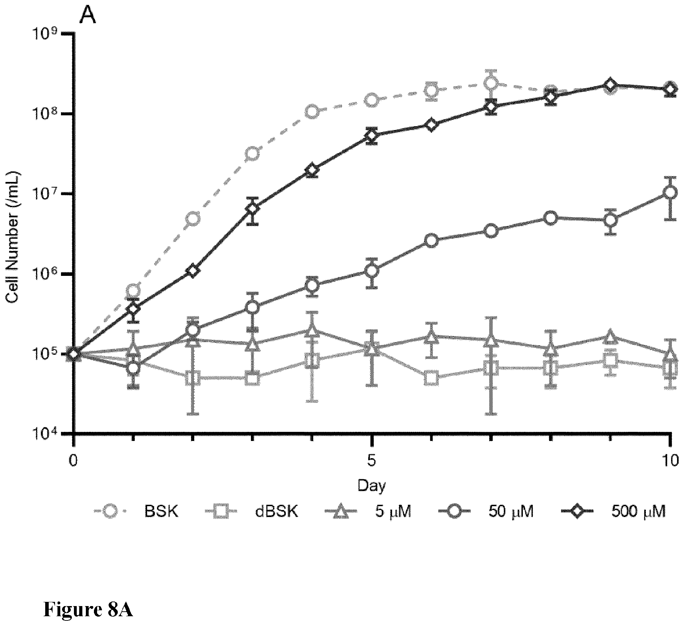

**Figure 8A****-****Figure 8B** are graphs demonstrating growth of *B. burgdorferi* is dependent upon exogenous lipids. A) Growth of B. burgdorferi in lipid-free medium supplemented with fatty acids and cholesterol (2:2: 1 palmitic acid: oleic acid: cholesterol) to a final concentration of 5-500 µM. At 500 µM, cell density equivalent to unmodified BSK medium is reached. No growth is observed in the absence of lipid (dBSK only). B) Equivalent growth over 10 days of *B. burgdorferi* in medium supplemented with fatty acids (FA: 200 µM each palmitic and oleic acids) and phospholipids (PL: 100 µM each phosphatidylglycerol and phosphatidylcholine). Cholesterol (Ch) was present at 100 µM in all media. Data plotted in both A and B are the mean of three biological replicates, with error bars showing standard deviation.

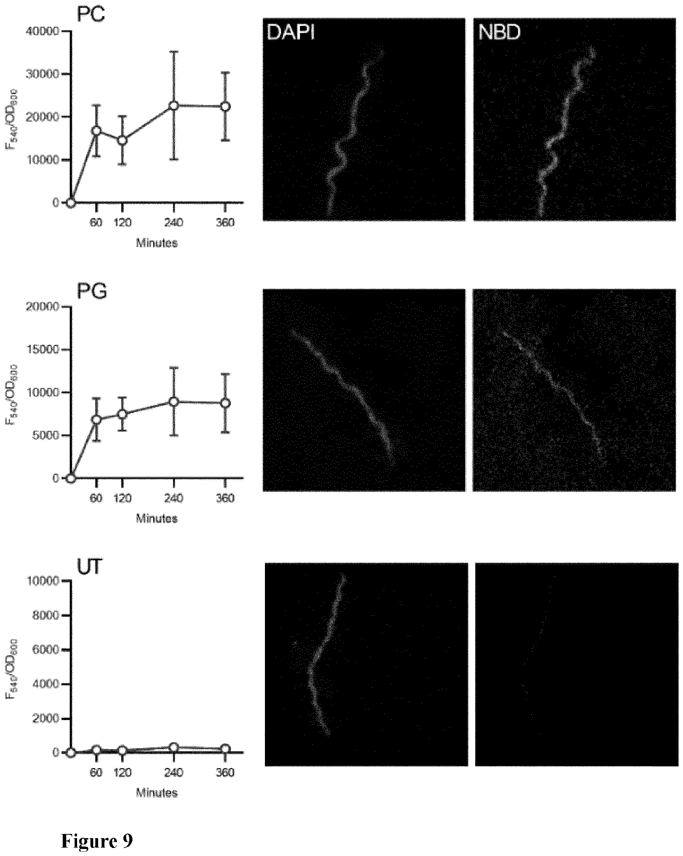

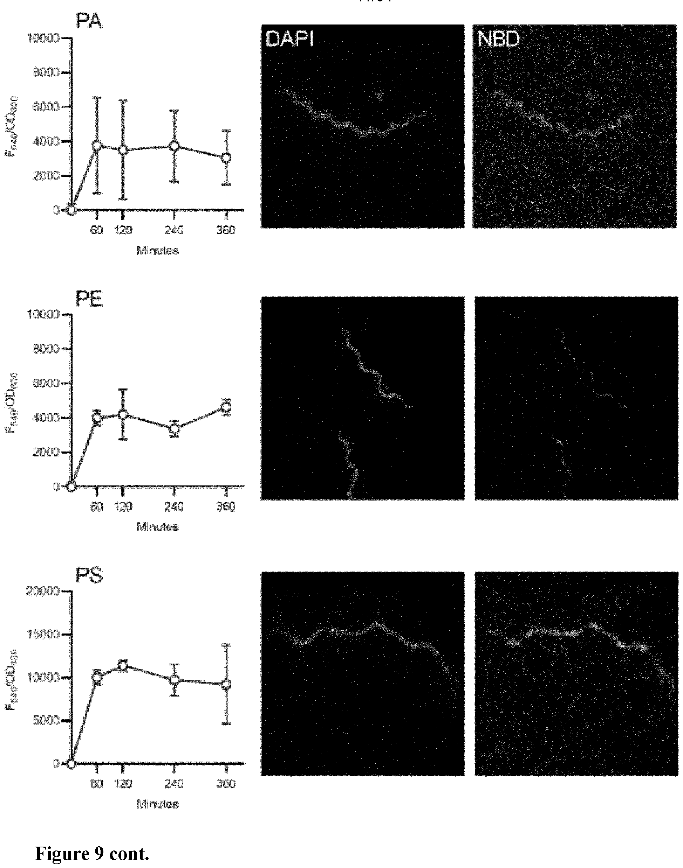

**Figure 9** includes graphs and fluorescent micrographs demonstrating uptake of fluorescent labelled phospholipids by *B. burgdorferi.* The canonical phospholipids of *B. burgdorferi* phosphatidylcholine (PC) and phosphatidylglycerol (PG) are acquired from the medium as well as the noncanonical membrane components phosphatidic acid (PA), phosphatidylethanolamine (PE), and phosphatidylserine (PS). Untreated cells (UT) do not fluoresce in the NBD channel. Cells were incubated in dBSK + 25 µM NBD-labelled phospholipids analogues over 6 hours, then imaged by fluorescence microscopy. DAPI stains nucleic acids nonspecifically. DAPI (Ex = 405 nm, detector = 410-466 nm) and NBD (Ex = 470 nm, detector 550-600 nm) channels were acquired sequentially. Fluorescence intensity (Ex = 465 nm, Em = 540 nm, normalized to OD600) was quantified by fluorimetry: graphs plot the mean of three biological replicates, with error bars the standard deviation. Images are representative of biological triplicate experiments.

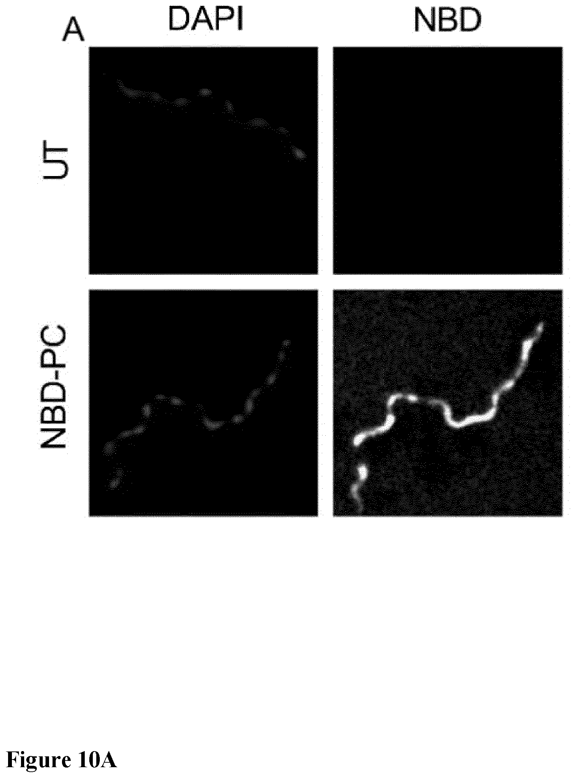

**Figures 10A****-****Figure 10C** includes fluorescence micrographs demonstrating that the membrane of *B. burgdorferi* is more accessible than that of other species. *B. burgdorferi* (A) *S. aureus* (B), *and E. coli* (C) were all incubated with 25 µM NBD-PC for 4 hours. Only *B. burgdorferi* acquires the fluorescent phospholipid. DAPI stains nucleic acids nonspecifically. DAPI (Ex = 405 nm, detector = 410-466 nm) and NBD (Ex = 470 nm, detector 550-600 nm) channels were acquired sequentially. Images are representative of biological triplicate experiments.

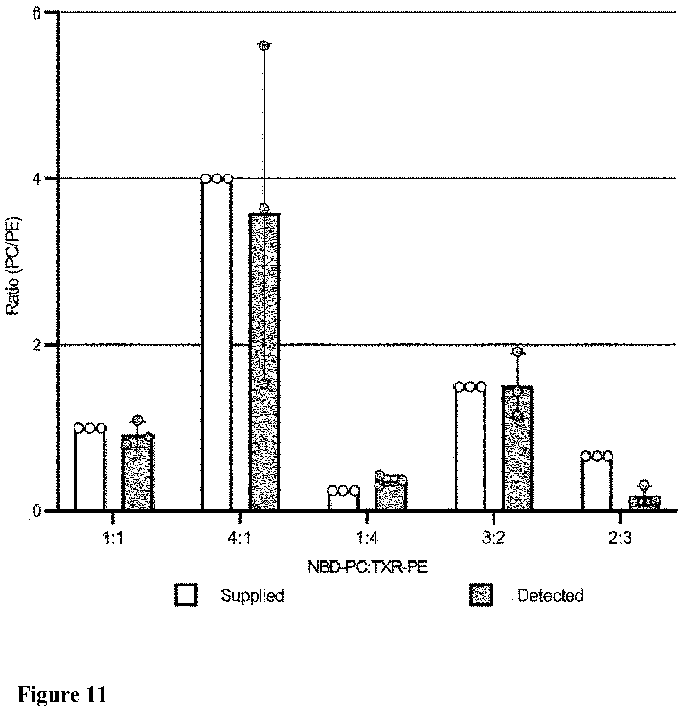

**Figure 11** is a graph demonstrating that the borrelial membrane reflects the surrounding medium. *B. burgdorferi* was incubated with two phospholipid fluorophores - NBBarsD-PC and Texas Red (TXR)-PE - at different ratios for four hours. The ratio of fluorescence intensity in washed cells (F540:F625) closely matches the ratio of fluorophore available in the culture medium (µM NBD-PC: µM TXR-PE). The total concentration of phospholipid was 25 µM at all conditions. Bars represent the mean of three biological replicates (dots), with error bars representing the standard deviation.

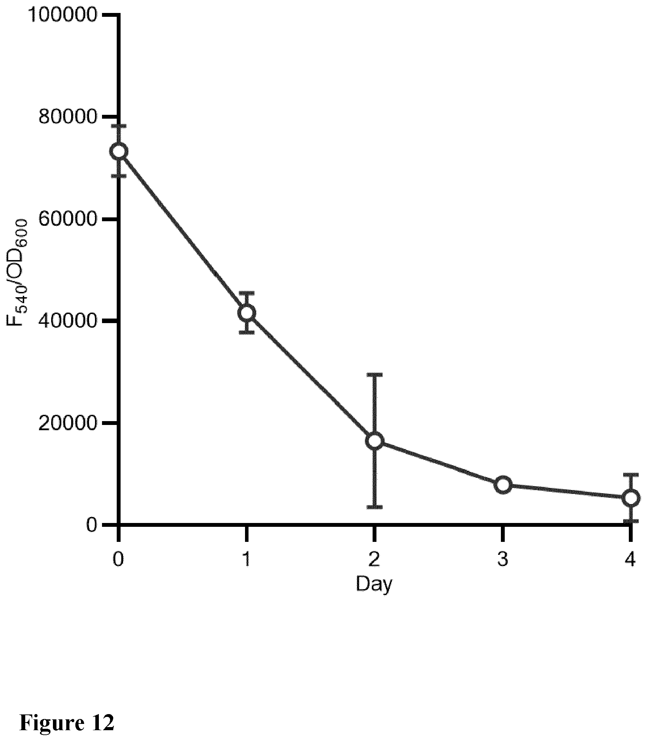

**Figure 12** is a graph illustrating that phospholipids are retained in the membrane in the absence of an environmental source. *B. burgdorferi* was labelled with NBD-PC in lipid-depleted dBSK medium for four hours, washed, and resuspended in rich medium. Fluorescence intensity (F540/OD600) was measured daily and declines with a half-life of ~24 hours, with 56% of the D0 fluorescence intensity at day 1 and ~7% at day 4. Points are the mean of three biological replicates, with error bars the standard deviation.

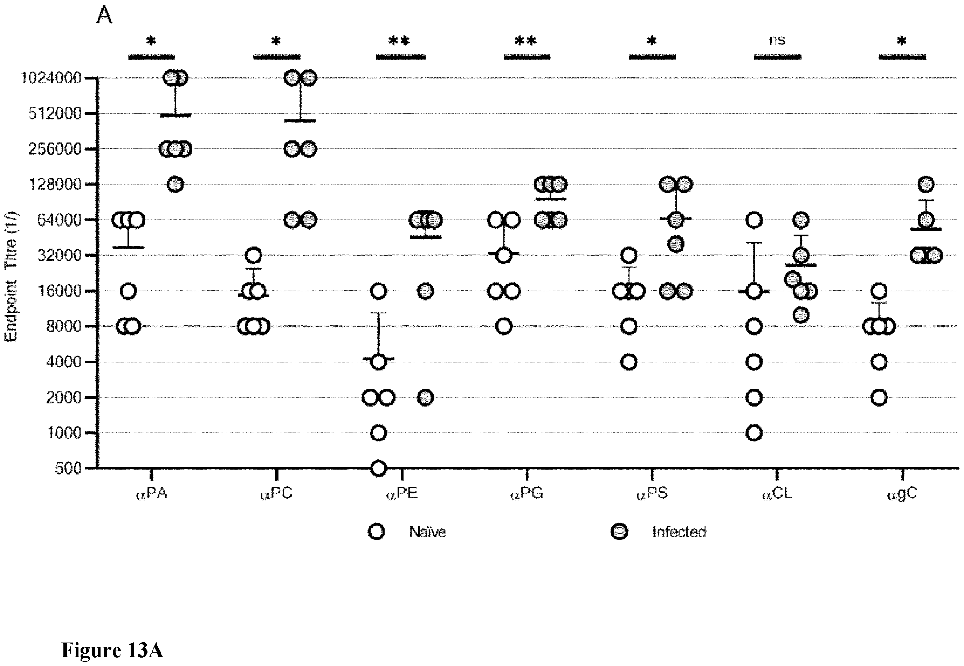

**Figures 13A****-****Figure 13B** are graphs demonstrating the presence of anti-lipid antibodies raised in a mouse model of *B. burgdorferi* infection. A) Endpoint titers were determined for seven antibodies (antiphosphatidic acid (αPA), antiphosphatidylcholine (αPC, antiphosphatidylethanolamine (αPE), antiphosphatidylglycerol (αPG), antiphosphatidylserine (αPS), anticardiolipin (αCL), antigalactosylcholesterol (αgC)) in naive and infected (4 week) C3H mice. Data (black lines indicate the mean of 6 replicates, with standard deviation as error bars) are plotted on a log2 scale to reflect binary dilution series. Significance was calculated by unpaired 2-tailed t test, where p >0.05 is nonsignificant. B) In a different group of 6 mice, three antibodies were measured over the course of a 28-day infection. Data plotted as A.

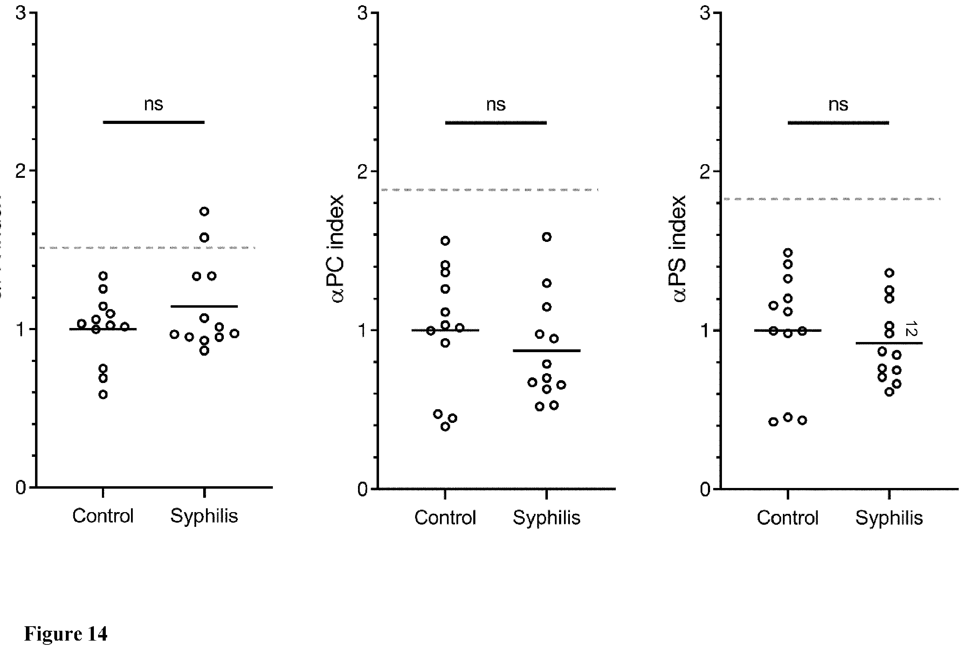

**Figure 14** includes graphs illustrating antiphospholipid indices in Syphilis (Treponema pallidum) infection. Twelve syphilis sera were tested for antiphosphatidic acid (αPA), antiphosphatidylcholine (αPC), and antiphosphatidylserine (αPS). None of the antibody titers were significantly different in syphilis and healthy control sera. Black lines represent the mean of each group. An index of >1 indicates antibody titer was above the level of the naive controls. Grey dashed lines represent the cutoff value (mean N + 2.291 standard deviations) above which a sample was considered positive. Significance calculated by unpaired 2-tailed t test, where p > 0.05 is nonsignificant (ns).

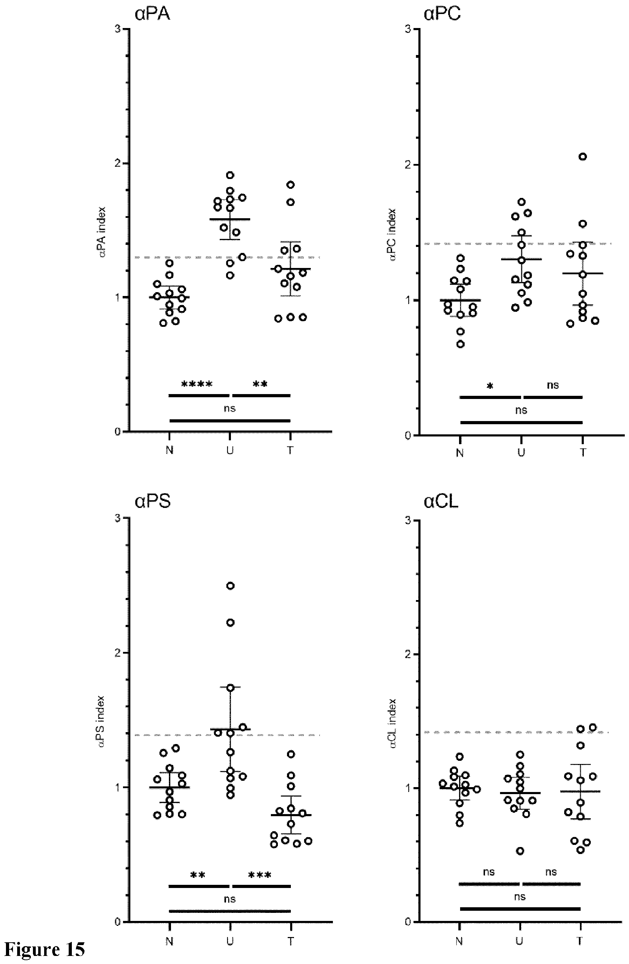

**Figure 15** includes graphs demonstrating anti-phospholipid antibodies in human sera during infection. IgG Antibodies to phosphatidic acid (αPA), phosphatidylcholine (αPC), phosphatidylserine (αPS) are raised during infection, while anticardiolipin (αCL) is not. Untreated (U) and post-treatment (T) groups were compared to naive controls (N). Each group contained 12 individuals, with black lines representing the mean of the group. All samples were normalized to the average of at least 6 naive controls run in parallel. An index of > 1 indicates antibody titer was above the level of the naive controls. Grey dashed lines represent the cutoff value (mean N + 2.291 standard deviations) above which a sample was considered positive. Significance calculated using Tukey's test for multiple comparisons, where p > 0.05 is nonsignificant (ns).

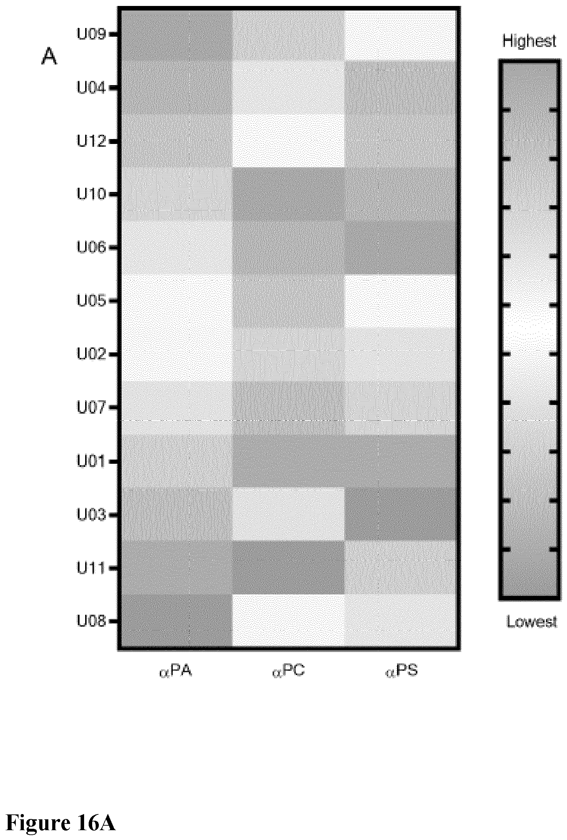

**Figures 16A****-****Figure 16D** includes graphs illustrating independence of the three antibody responses. A) heat map showing rank score for each elevated antibody in each serum sample. B-D) Linear regression for pairwise comparison of the three antibody responses. Only αPC vs. αPS is significant by unpaired 2-tailed t test (p < 0.05). Trend lines shown in black and 5/95% confidence intervals in grey dashes.

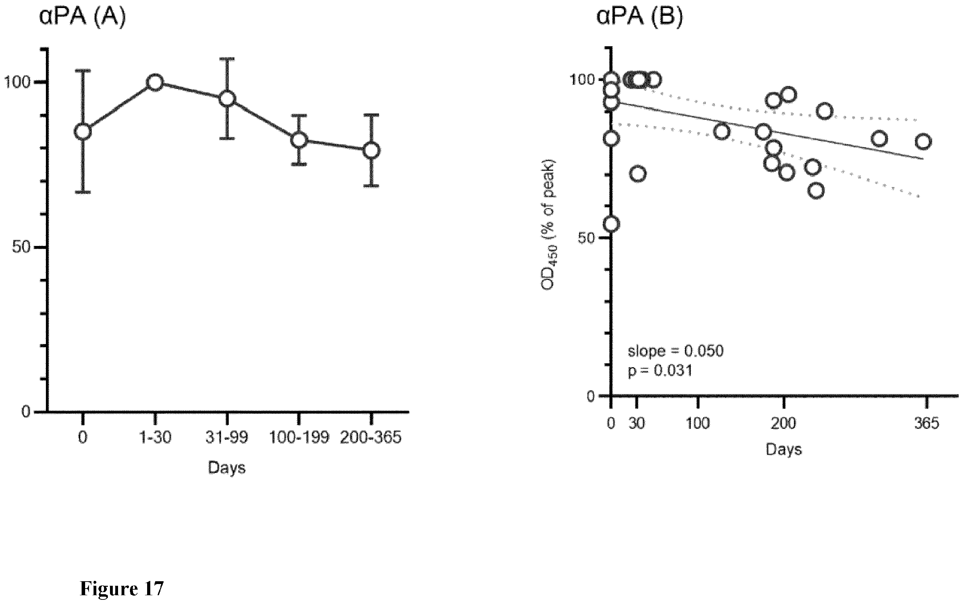

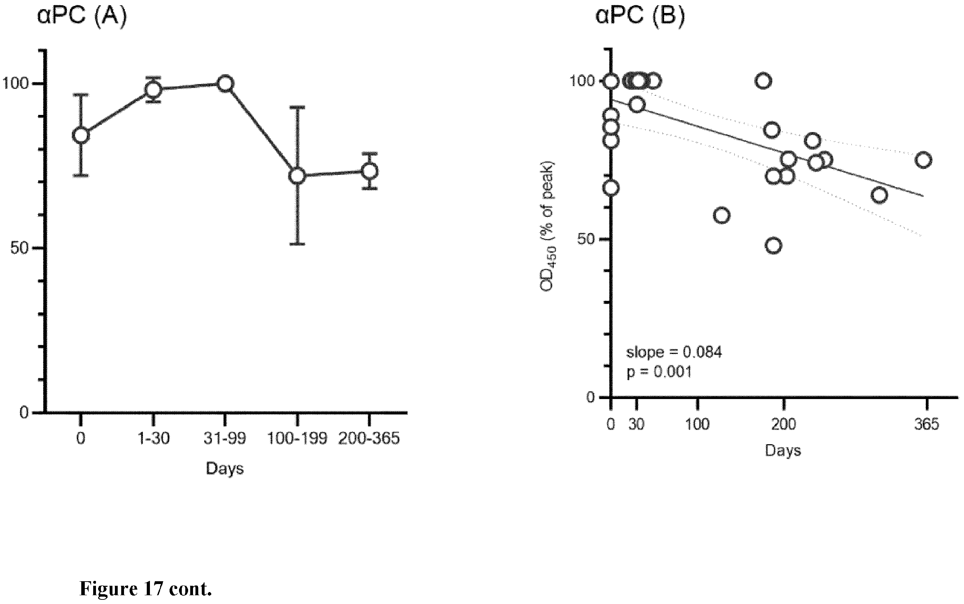

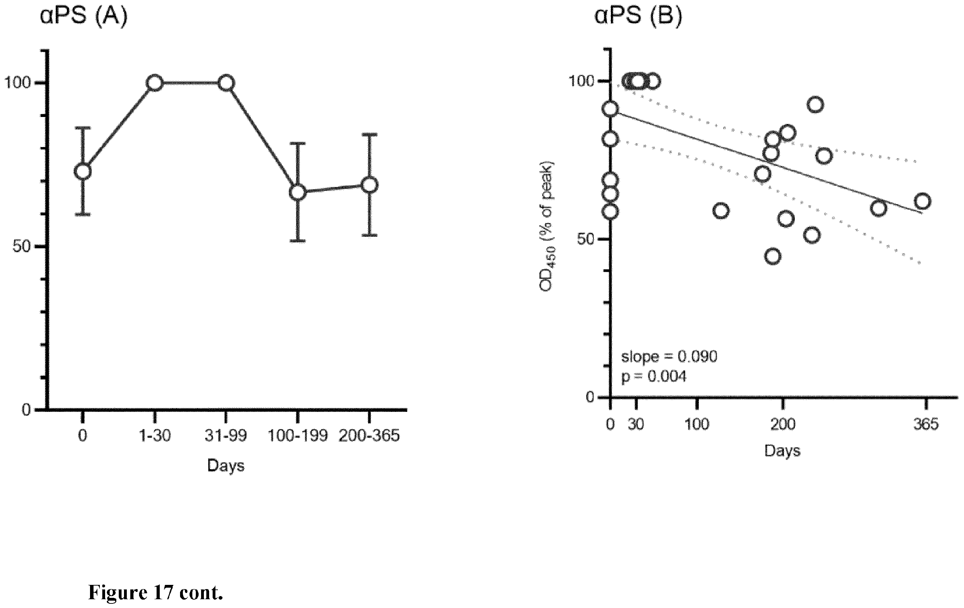

**Figure 17** includes graphs illustrating the decline of antibody titers after treatment. A group of ten individuals were sampled serially (2, 3, or 4 times) at time points 0-365 days from the beginning of treatment. Antibody titers (αPA, αPC, and αPS) for each patient are shown in as percentages of the highest value for each patient/antibody. In (A) samples are grouped by time point, with n = 5 at D0, 4 at D1-30, 6 at D31-99, 5 at D100-199, and 7 at D200-365. Error bars represent the standard deviation. In (B) samples are plotted individually against day of collection, with trend lines shown in black and 5/95% confidence intervals in grey dashes.

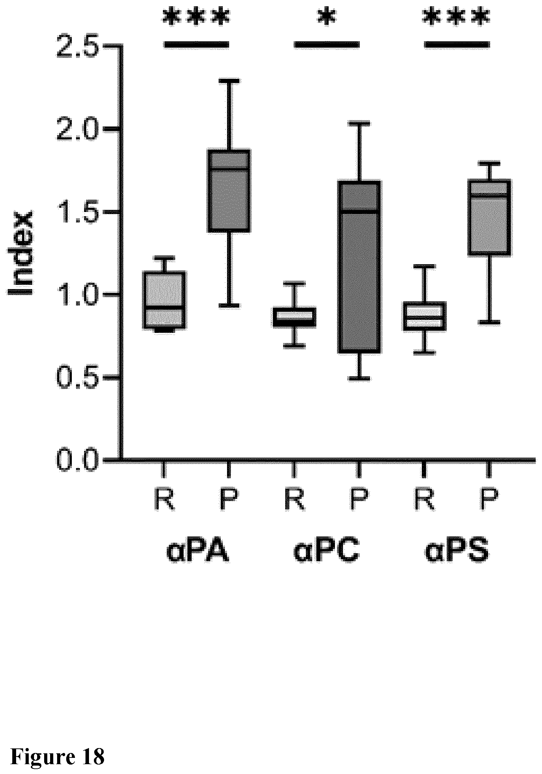

**Figure 18** is a graph illustrating antibody (indices) for post-treatment patients, where a value of 1.0 is the same as healthy controls. Patients whose symptoms are resolved (R) return to levels similar to the controls, where some of those with persistent symptoms (P) remain elevated.

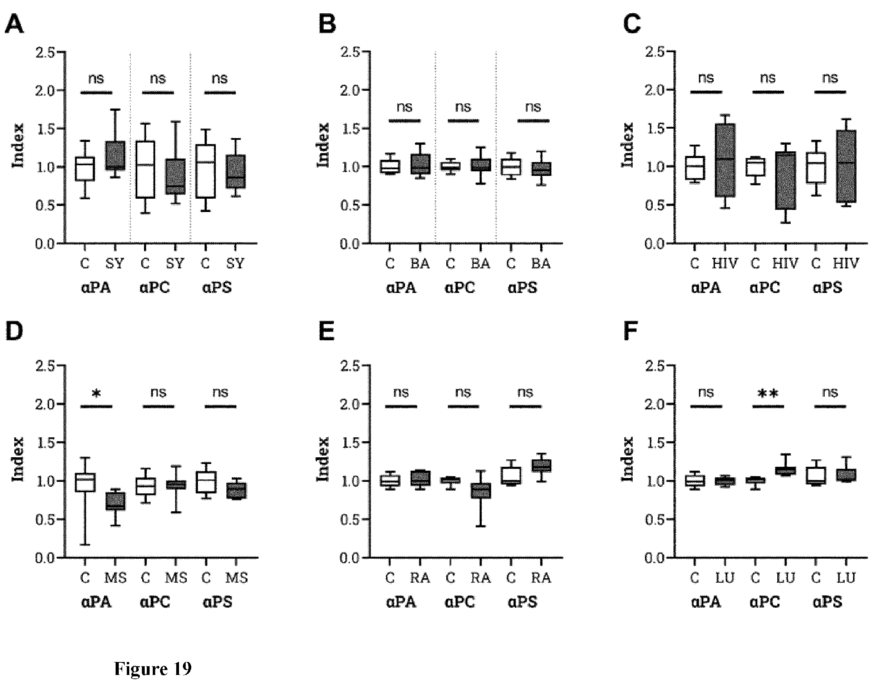

**Figures 19A****-****Figure 19F** includes graphs illustrating antibody levels (indices) in healthy controls (C) and other infectious or autoimmune conditions. A) Syphilis; SY. B) Babesiosis; BA. C) HIV. D) Multiple sclerosis; MS. E) Rheumatoid arthritis; RA. F) Systemic lupus erythromatous; LU. One antibody is significantly lower in MS than controls, and one is higher than controls in lupus. Groups of 10 or 12 patients.

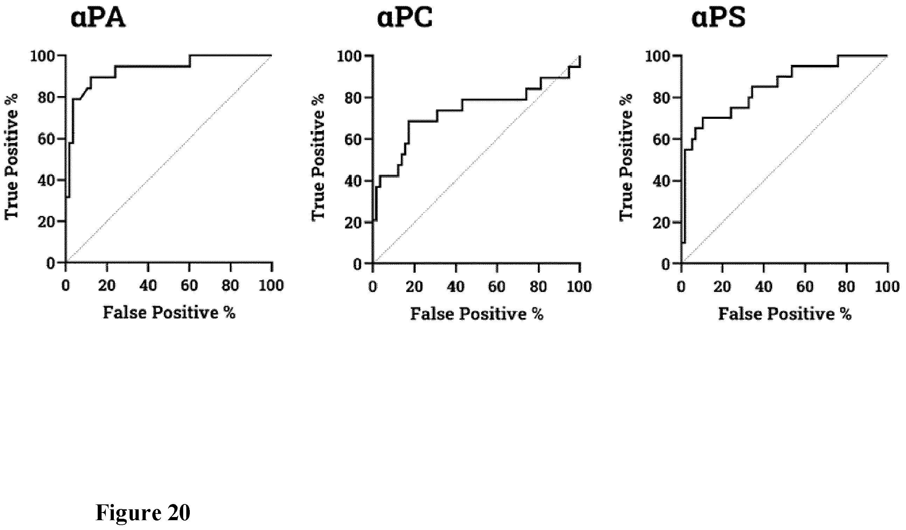

**Figure 20** includes graphs illustrating receiver operating characteristic (ROC) curves that describe the effectiveness of using antiphospholipids (aPA, aPC, aPS) to distinguish Lyme infections (true positives) from healthy or other disease controls (false positives).

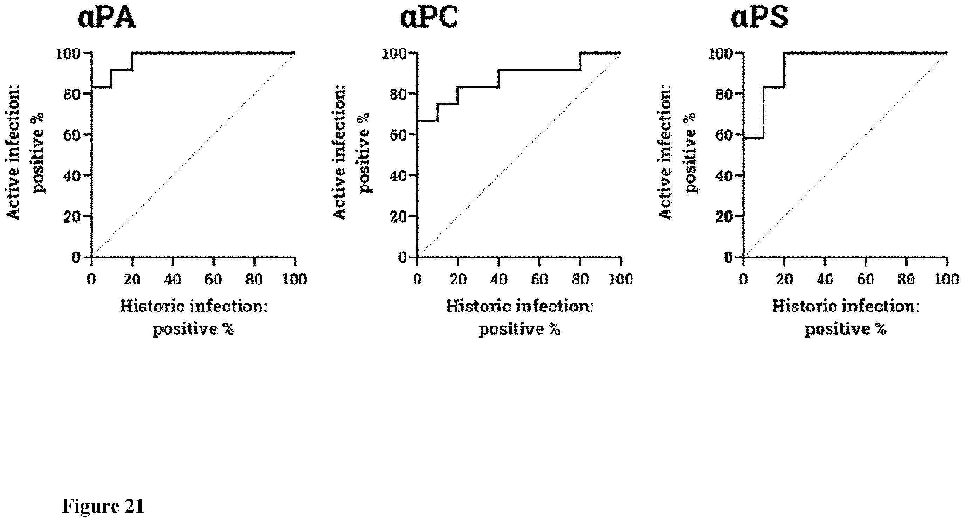

**Figure 21** includes graphs illustrating ROC curves that describe the effectiveness of using antiphospholipids (aPA, aPC, aPS) to active Lyme infections from treated, historic infections.

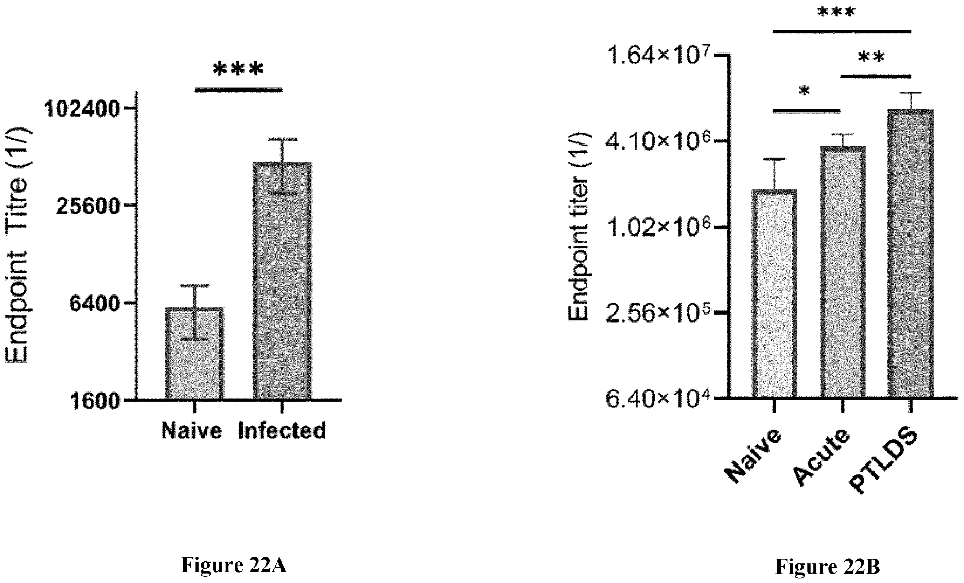

**Figure 22A** is a graph illustrating the elevation of anticeramide in infected mice vs. control mice. 6 mice per group, infection duration: 4 weeks.
**Figure 22B** is a graph illustrating the elevation of antisphingomyefin in acute infections and further elevated in persistent Lyme disease (PTLDS). Groups of 6 patients.

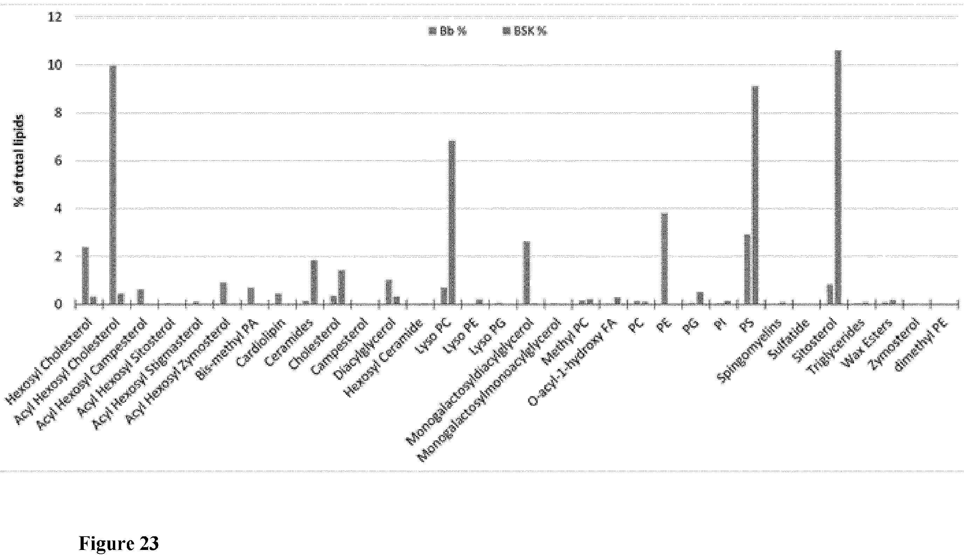

**Figure 23** is a graph illustrating lipids detected in *B. burgdorferi* membranes by mass spectrometry. The lipid content of cell membranes (Bb) were compared to the lipid content of the standard growth medium (BSK).

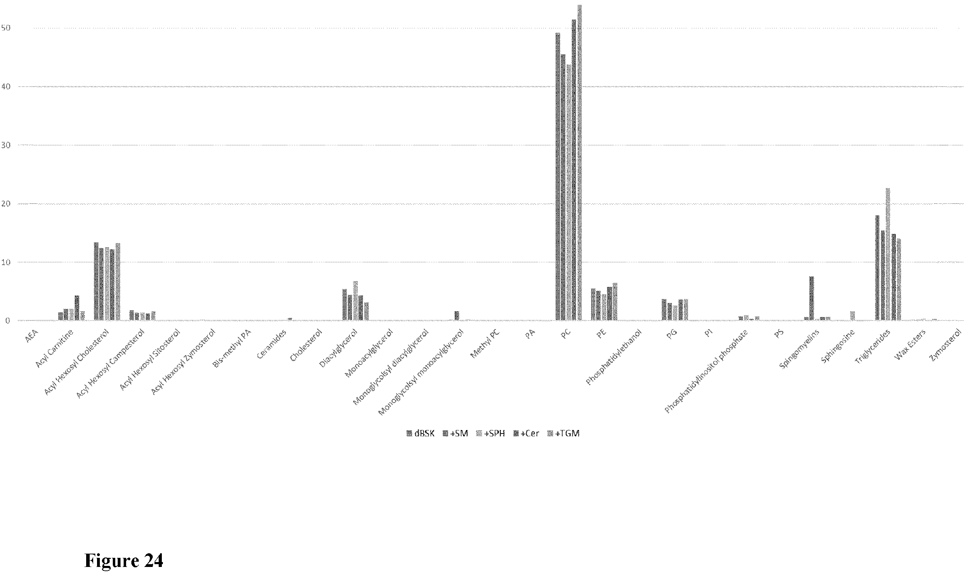

**Figure 24** is a graph illustrating lipids detected in *B. burgdorferi* membranes by mass spectrometry. In this experiment, *B. burgdorferi* was cultured in a non-standard growth medium (dBSK) supplemented with lipids of various types.

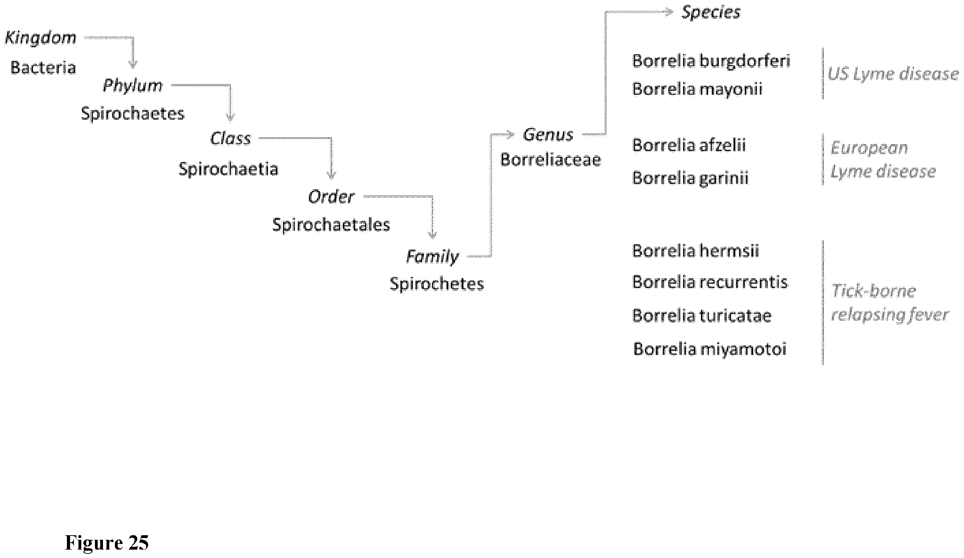

**Figure 25** is a flowchart illustrating the taxonomy of *Borreliaceae* species.

### DETAILED DESCRIPTION

Before explaining one or more embodiments of the disclosure in detail, it is to be understood that the embodiments are not limited in their application to the details of construction and the arrangement of the components or steps or methodologies set forth in the following description or illustrated in the drawings. In the following detailed description of embodiments, numerous specific details may be set forth in order to provide a more thorough understanding of the disclosure. However, it will be apparent to one of ordinary skill in the art having the benefit of the instant disclosure that the embodiments disclosed herein may be practiced without some of these specific details. In other instances, well-known features may not be described in detail to avoid unnecessarily complicating the instant disclosure.

As used herein a letter following a reference numeral is intended to reference an embodiment of the feature or element that may be similar, but not necessarily identical, to a previously described element or feature bearing the same reference numeral (e.g., 1, 1a, 1b). Such shorthand notations are used for purposes of convenience only and should not be construed to limit the disclosure in any way unless expressly stated to the contrary.

Further, unless expressly stated to the contrary, "or" refers to an inclusive or and not to an exclusive or. For example, a condition A or B is satisfied by anyone of the following: A is true (or present) and B is false (or not present), A is false (or not present) and B is true (or present), and both A and B are true (or present).

In addition, use of "a" or "an" may be employed to describe elements and components of embodiments disclosed herein. This is done merely for convenience and "a" and "an" are intended to include "one" or "at least one," and the singular also includes the plural unless it is obvious that it is meant otherwise.

Finally, as used herein any reference to "one embodiment" or "some embodiments" means that a particular element, feature, structure, or characteristic described in connection with the embodiment is included in at least one embodiment disclosed herein. The appearances of the phrase "in some embodiments" in various places in the specification are not necessarily all referring to the same embodiment, and embodiments may include one or more of the features expressly described or inherently present herein, or any combination of subcombination of two or more such features, along with any other features which may not necessarily be expressly described or inherently present in the instant disclosure.

A close association with its vertebrate and tick hosts allows *Borrelia burgdorferi,* the bacterium responsible for Lyme disease, to eliminate many metabolic pathways and instead scavenge key nutrients from the host. A novel lipid-defined culture medium was used to demonstrate that exogenous lipids are an essential nutrient of *B. burgdorferi.* Intact phospholipids are also acquired from the growth medium, can support growth, and enter the membrane directly. Several of these environmentally acquired phospholipids are the targets of antibodies that arise during the course of infection in both a mouse model and in Lyme disease patients. In a cohort of serum samples taken from patients with Lyme disease, the elevation of antiphospholipid antibodies predicts infection with a similar sensitivity to the standardized two-tier tests currently used in diagnosis. Antiphospholipid tests also show marked declines in antibody titer after antibiotic treatment, potentially allowing quantitation of the resolution of disease.

The inventors have identified three classes of antibody biomarkers which are elevated during infection by *Borrelia burgdorfei*, one of the bacteria which causes Lyme disease. The three classes of antibodies are antiphospholipid antibodies which bind (1) phosphatidic acid (PA), (2) phosphtidylcholine (PC), and (3) phosphatidylserine (PS). These antibodies arise earlier than other antibody markers currently used for the clinical diagnosis of Lyme disease and so may allow for improved detection in the first two weeks of infection.

The antiphospholipid antibody levels for antibodies in these three classes also return to baseline faster than current diagnostic antibodies. By conventional testing based on different antibody markers, many people remain positive for decades after the infection is resolved, which prohibits repeat testing in individuals with a history of Lyme disease. Antibodies that decline more rapidly allow for repeated testing an also for antibody tests that can monitor the success or failure of treatment. The CDC specifically advises against us of the current test for these applications.

In illustrative embodiments, the inventors determined antiphospholipid antibody levels using ELISA, a common laboratory technique widely used in clinical diagnostics. In principle, antiphospholipid antibodies levels could also be determined by other immunoassays such as immunofluorescence.

The inventors have also collected patient data for the three types of antibodies binding the following targets antiphosphatidic acid, antiphosphtidylcholine, and antiphosphatidylserine. The broad class of antiphospholipid antibodies contains other members. Of these, two more types of antiphospholipid antibodies were found to be elevated in a mouse model of infection. These two types, antiphosphatidylglycerol and antiphosphatidylethanolamine antibodies, may also be found to be elevated in human infection by *Borrelia burgdorferi* or *Borrelia mayonii.* In addition, in some embodiments, antibodies to other lipids found to be incorporated in the *Borrelia sp*., membrane, such as sphingolipids, may be found to be elevated in human infection by a *Borrelia* sp. Thus, antibody levels of antibodies to phosphatidylglycerol, phosphatidylethanolamine, and/or certain other lipids and related compounds may also be useful as diagnostic markers. By way of example but not by way of limitation, the inventors have determined that antibodies against hexosyl cholesterol, acyl hexosyl cholesterol, among other compounds, result from infection by *Borrelia* sp. and provide a diagnostic tool to identify and monitor *Borrelia sp.* infection and treatment.

Illustrative, non-limiting advantages of the current technology include improved performance for patients, simpler result for physicians to interpret, easier testing methodologies for clinical staff, reduction in the use of antibiotics to treat infection, and kits for the convenience of clinical or at-home diagnostic testing.

### Definitions and Terminology

The disclosed compositions, system, kits and methods for detecting and diagnosing infection by a Borrelia *sp*., such as but not limited to *Borrelia afzelii*, *Borrelia bissettiae, Borrelia burgdorferi, Borrelia garinii, Borrelia lusitaniae, Borrelia mayonii, Borrelia spielmanii*, and *Borrelia valaisiana.* may be further described using definitions and terminology as follows. The definitions and terminology used herein are for the purpose of describing particular embodiments only and are not intended to be limiting.

As used in this specification and the claims, the singular forms "a," "an," and "the" include plural forms unless the context clearly dictates otherwise.

As used herein, "about", "approximately," "substantially," and "significantly" will be understood by persons of ordinary skill in the art and will vary to some extent on the context in which they are used. If there are uses of the term which are not clear to persons of ordinary skill in the art given the context in which it is used, "about" and "approximately" will mean up to plus or minus 10% of the particular term and "substantially" and "significantly" will mean more than plus or minus 10% of the particular term.

As used herein, the terms "include" and "including" have the same meaning as the terms "comprise" and "comprising." The terms "comprise" and "comprising" should be interpreted as being "open" transitional terms that permit the inclusion of additional components further to those components recited in the claims. The terms "consist" and "consisting of" should be interpreted as being "closed" transitional terms that do not permit the inclusion of additional components other than the components recited in the claims. The term "consisting essentially of" should be interpreted to be partially closed and allowing the inclusion only of additional components that do not fundamentally alter the nature of the claimed subject matter.

The phrase "such as" should be interpreted as "for example, including." Moreover, the use of any and all exemplary language, including but not limited to "such as", is intended merely to better illuminate the invention and does not pose a limitation on the scope of the invention unless otherwise claimed.

Furthermore, in those instances where a convention analogous to "at least one of A, B and C, etc." is used, in general such a construction is intended in the sense of one having ordinary skill in the art would understand the convention (e.g., "a system having at least one of A, B and C" would include but not be limited to systems that have A alone, B alone, C alone, A and B together, A and C together, B and C together, and/or A, B, and C together.). It will be further understood by those within the art that virtually any disjunctive word and/or phrase presenting two or more alternative terms, whether in the description or figures, should be understood to contemplate the possibilities of including one of the terms, either of the terms, or both terms. For example, the phrase "A or B" will be understood to include the possibilities of "A" or 'B or "A and B."

All language such as "up to," "at least," "greater than," "less than," and the like, include the number recited and refer to ranges which can subsequently be broken down into ranges and subranges. A range includes each individual member. Thus, for example, a group having 1-3 members refers to groups having 1, 2, or 3 members. Similarly, a group having 6 members refers to groups having 1, 2, 3, 4, or 6 members, and so forth.

The modal verb "may" refers to the preferred use or selection of one or more options or choices among the several described embodiments or features contained within the same. Where no options or choices are disclosed regarding a particular embodiment or feature contained in the same, the modal verb "may" refers to an affirmative act regarding how to make or use and aspect of a described embodiment or feature contained in the same, or a definitive decision to use a specific skill regarding a described embodiment or feature contained in the same. In this latter context, the modal verb "may" has the same meaning and connotation as the auxiliary verb "can."

"Lyme disease" as used herein refers to an infection by a *Borrelia* bacterium, which is also known as Borreliosis. At least eight species of *Borrelia* are known to cause Lyme disease, including *Borrelia afzelii*, *Borrelia bissettiae, Borrelia burgdorferi*, *Borrelia garinii*, *Borrelia lusitaniae, Borrelia mayonii*, *Borrelia spielmanii*, and *Borrelia valaisiana.* Lyme disease is commonly caused by the transfer of Borrelia bacteria to mammalian hosts by certain hard ticks of the *Ixodidae* family. In the U.S., Lyme disease is most commonly the result of the transfer of *Borrelia burgdorferi* bacteria by the blacklegged tick, also known as a deer tick (*Ixodes scapularis* or *Ixodes pacificus*)*.* Outside the US, *Ixodes ricinus* and *Ixodes persulcatus* act as Borreliosis vectors (e.g., for *B. afzelii* or *B. garinii*)*.*

Without early detection and treatment, the bacteria travel through the bloodstream and affect various tissues in the host. The infection may develop into an inflammatory condition that affects multiple systems, starting with skin, joints, and nervous system and moving to organs. Symptoms can include one or more of fever, chills, headache, fatigue, muscle pain, joint pain, swollen lymph nodes, a bullseye rash, termed erythema migrans, facial palsy, and arthritis. Symptoms of early and late Lyme disease are demonstrated in Figures 2 and 3, respectively.

The term "subject" may be used interchangeably with the terms "individual" and "patient" and includes human and non-human subjects. In some embodiments, subjects may be any animal that can be infected with a bacterium, such as *Borrelia burgdorferi.* In some embodiments, the subject is a mammal, such as a human, dog, cat, or livestock, such as cattle, pigs, or sheep. In some embodiments, a subject may include a wild, domesticated, or captive population of animals such as deer, elk, bison, etc. In some embodiments, a subject may include one or more wild or domesticated bird species, such as chickens, ducks, or turkeys.

In some embodiments, the methods include detecting the presence of *Boriellia sp.* in a subject, such as but not limited to *Borrelia afzelii, Borrelia burgdorferi, Borrelia garinii,* or *Borrelia mayonii.*

As used herein, the terms "treat" or "treatment" encompass both "preventative" and "curative" treatment. "Preventative" treatment is meant to indicate a postponement of development of a disease, a symptom of a disease, or medical condition, suppressing symptoms that may appear, or reducing the risk of developing or recurrence of a disease or symptom. "Curative" treatment includes reducing the severity of or suppressing the worsening of an existing disease, symptom, or condition. Thus, treatment includes ameliorating or preventing the worsening of existing disease symptoms, preventing additional symptoms from occurring, ameliorating or preventing the underlying systemic causes of symptoms, inhibiting the disorder or disease, e.g., arresting the development of the disorder or disease, relieving the disorder or disease, causing regression of the disorder or disease, relieving a condition caused by the disease or disorder, or stopping the symptoms of the disease or disorder.

A non-limiting example of a treatment for infection by *Borrelia sp.* (*e.g*., Lyme disease) includes, but is not limited to, antibiotic administration.

A non-limiting example of a treatment for a subject diagnosed with Lyme disease includes, but is not limited to, antibiotic administration.

As used herein, the term "antibody," or "antibody molecule" (used synonymously herein) refer to naturally occurring immunoglobulin molecules with varying structures. Typically, antibodies are gamma globulin proteins that can be found in blood or other bodily fluids of vertebrates and are used by the immune system to identify foreign materials, such as bacteria, viruses, and toxins. Antibodies bind, by non-covalent interactions, with high affinity to other molecules or structures known as antigens. This binding is specific in the sense that an antibody molecule will only bind to a specific structure with high affinity. The unique part of the antigen recognized by an antibody molecule is called an epitope, or antigenic determinant. The part of the antibody molecule binding to the epitope is sometimes called paratope and resides in the so-called variable domain, or variable region (Fv) of the antibody. The variable domain comprises three complementary-determining regions (CDR's) spaced apart by framework regions (FR's). There are at least five major classes of antibodies: IgA, IgD, IgE, IgG, and IgM, and several of these may be further divided into subclasses (isotypes), e.g. , IgG1, IgG2, IgG3, IgG4, IgA1, and IgA2. They are typically made of basic structural units--each with two large heavy chains and two small light chains--to form, for example, monomers with one unit, dimers with two units or pentamers with five units. Non-limiting examples of antibodies include any known class and/or isotype, such as, e.g., IgA (e.g. IgA1, IgA2, and sIgA), IgD, IgE, IgG (e.g. IgG1, IgG2, IgG3, or IgG4), and IgM. The "class" of an antibody may refer to the type of constant domain or constant region possessed by its heavy chain. For example, heavy chain constant domains may be used to classify different classes of immunoglobulins, such as, e.g., α, γ, δ, ε, and µ. In some embodiments, the antibody is a native human IgG. IgG antibodies are typically heterotetrameric glycoproteins of about 150,000 Daltons, composed of two identical light chains and two identical heavy chains that are disulfide bonded. The light chain of an antibody may be assigned to certain types, e.g. kappa and lambda, based on the amino acid sequence of its constant domain.

The terms "epitope" and "antigenic determinant", which can be used interchangeably, refer to the part of a macromolecule, such as a polypeptide, carbohydrate, or a lipid, that is recognized by antigen-binding molecules, such antibodies. Epitopes define the minimum binding site for an antibody molecule, and thus represent the target of specificity of an antibody molecule.

An antibody molecule that can "bind", "bind to", "specifically bind", or "specifically bind to", that "has affinity for" and/or that "has specificity for" a certain epitope, antigen or protein (or for at least one part, fragment or epitope thereof) is said to be "against" or "directed against" said epitope, antigen or protein or is a "binding" molecule with respect to such epitope, antigen or protein.

Generally, the term "specificity" refers to the number of different types of antigens or epitopes to which a particular antigen-binding molecule or antigen-binding protein (such as an immunoglobulin, an antibody, an immunoglobulin single variable domain) can bind. The specificity of an antigen-binding protein can be determined based on its affinity and/or avidity. The affinity, represented by the equilibrium constant for the dissociation of an antigen with an antigen-binding protein (K_{D}), is a measure for the binding strength between an epitope and an antigen-binding site on the antigen-binding protein: the lesser the value of the K_{D}, the stronger the binding strength between an epitope and the antigen-binding molecule (alternatively, the affinity can also be expressed as the affinity constant (K_{A}), which is 1/K_{D}). As will be clear to the skilled person (for example on the basis of the further disclosure herein), affinity can be determined in a manner known per se, depending on the specific antigen of interest. Avidity is the measure of the strength of binding between an antigen-binding molecule (such as an antibody of the invention) and the pertinent antigen. Avidity is related to both the affinity between an epitope and its antigen binding site on the antigen-binding molecule and the number of pertinent binding sites present on the antigen-binding molecule.

As used herein, the term "panel" refers to a collection of two or more biomolecules (e.g. antigens) wherein the two or more biomolecules are organized such that the two or more biomolecules can be differentiated from each other in a detecting step. This organization can be a spatial arrangement (e.g. biomolecule 1 is in section 1 of the panel, biomolecule 2 is in section 2 of the panel, etc.) or a difference in a detection aspect, such as each biomolecule can be differentiated by a different detectable agent (e.g. biomolecule 1 can be detected with color 1, biomolecule 2 can be detected with color 2, etc.).

As used herein, the term "solid support" refers to any substrate or support matrix to which a molecule (such as a lipid, antibody, or antibody-lipid complex) can be bound, either reversibly or irreversibly. Non-limiting examples of solid supports include, without limitation, a bead, plate, slide, flow chamber, test strip, chip, cartridge, flow cell, etc. The solid support may be formed from glass, ceramic, polymers (e.g., plastic, latex, polystyrene, polyacrylamide, polyvinylchloride, polypropylene, polyethylene, polylactic acid), cellulose (e.g., paper), n some embodiments, a lipid is immobilized on a solid support. In some embodiments, the lipid is a phospholipid. The lipid may be phosphatidic acid (PA), phosphatidyl choline (PC), and/or phosphatidylserine (PS), and optionally one or more of at least one of ceramide, sphingomyelin, hexosyl cholesterol, acyl hexosyl cholesterol, galactosyldiacylglycerol, or phosphatidylethanolamine. In some embodiments, the immobilized lipid is bound to the solid support, either reversibly or irreversibly, to form a platform for an antibody binding reaction. In some embodiments, there are two or more lipids which are immobilized on the solid support in configuration so as to form a lipid panel.

As used herein, the term "bead" refers to a microbead or relatively small bead having a diameter less than about 500 µm, typically with a diameter of less than about 100 µm, 50 µm, or 10 µm. While a bead may vary in shape, typically a bead is substantially spherical, e.g. a microsphere. Microbeads are known in the art and can comprise various materials such as, e.g., latex, polystyrene, polyacrylamide, polyvinylchloride, polypropylene, polyethylene, polylactic acid, ceramic, glass, and magnetic compositions.

As used herein, the term "plate" encompasses a solid support comprising a variety of types, including plastic or glass plates. In some embodiments, the plate is a 96-well, 384-well, or 1536-well plastic plate.

As used herein, a "detectable binding agent" refers to a molecule that binds, either directly or indirectly, to a target molecule, and can be detected either directly, or with further chemical or enzymatic reaction. By way of example, but not by way of limitations, a detectable binding agent may comprise an antibody linked to an enzyme, such as horseradish peroxidase (HRP). Addition of an HRP substrate, under enzymatic reaction conditions, will allow detection of the HRP-bound molecule and the target. Other examples of detectable binding agents and include antibodies linked to detectable labels, such as fluorescent labels. Such constructs are well known in the art and are not intended to limit the scope of the present methods and compositions.

As used herein, a "detectable agent" or "detectable label" refers to an agent or label that can be detected either directly, or with further chemical, reagent, or enzymatic reaction. By way of example, but not by way of limitations, a detectable agent or label may comprise a fluorescent moiety or an enzyme, such as HRP. Other examples of detectable agents and labels include radioisotope. Such agents and labels are well known in the art and are not intended to limit the scope of the present methods and compositions.

As used herein, the term "buffer" refers to a pH buffering agent that helps maintain the pH of an aqueous solution. Non-limiting examples of pH buffering agents include phosphate buffers (e.g. PBS), Tris, citric acid, histidine, glycine, HEPES, MOPS, and PIPES.

As used herein, the term "stabilizer" refers to an agent that stabilizes a component of a composition, such as a protein, antibody, and/or lipid, thereby helping to preserve its stability and integrity. Non-limiting examples of stabilizers include glycerol, polysorbates, polyethylene glycols (PEGs), glycine, histidine, arginine, sugars (e.g. trehalose), and polyols (e.g., mannitol, sorbitol and glycerol).

As used herein, the term "non-specific binding surface blocking agent" refers to an agent that reduces the non-specific binding between antibodies and other molecules. Non-limiting examples of non-specific binding surface blocking agents include milk proteins (e.g. casein) and bovine serum albumin (BSA).

As used herein, the term "macromolecular crowding agent" refers to an inert agent that excludes volume in solution from other macromolecules, such as proteins and antibodies. Non-limiting examples of macromolecular crowding agents include polyethylene glycols (PEGs), dextran, and high molecular weight, highly branched polysaccharides.

As used herein, the term "antioxidant" refers to an agent that inhibits oxidation reactions. Non-limiting examples of antioxidants include citrate, EDTA (ethylenediaminetetraacetic acid), methionine, ascorbic acid, and thiols.

As used herein, the term "preservative" refers to an agent that prevents or inhibits microbiological growth. Non-limiting examples of preservatives include CMIT (5-chloro-2-methylisothiazol-3(2H)-one), MIT (2-methylisothiazol-3(2H)-one), and sodium azide.

As used herein, the term "surfactant" refers to an agent that modulates the solubility of another molecule or agent in a solution. Non-limiting examples of surfactants include polysorbate 20, polysorbate 80, Triton X-100, and pluronic F68.

As used herein, the term "cut-off value" refers to a dividing point for a quantitative result of a diagnostic test or screen to be indicative of a positive or negative result based on measurements of the level of a molecular marker(s). This disclosure provides methods of detecting Lyme disease and/or *Borrelia* infection in a subject comprising the step of comparing a measured level of molecular marker to a predetermined value. In some embodiments, comparing a measured value of an antiphospholipid antibody level in a sample to a predetermined cut-off value may be used to decide whether the method indicates a positive or negative result of Lyme disease and/or *Borrelia* infection in the subject. As used in the claims, a "cut-off value" refers to a value above which a measured antiphospholipid antibody level in a sample is indicative of the positive presence of a *Borrelia* infection and/or Lyme disease in the subject from which the sample was derived.

As used herein the term "standard two-tier test" (SST) refers to a decision tree that describes the steps required to properly test for Lyme disease. The first required test is the Enzyme Immunoassay (EIA) or Immunofluorescence Assay (IFA). If this test yields negative results, the provider should consider an alternative diagnosis; or in cases where the patient has had symptoms for less than or equal to 30 days, the provider may treat the patient and follow up with a convalescent serum. If the first test yields positive or equivocal results, two options are available: 1) If the patient has had symptoms for less than or equal to 30 days, an IgM Western Blot is performed; 2) if the patient has had symptoms for more than 30 days, the IgG Western Blot is performed. The IgM should not be used if the patient has been ill for more than 30 days. A diagram of the SST decision tree is shown in **Figure 4****.**

### Compositions

Disclosed herein are compositions useful for the detection of infection with *Borrelia sp.* (e.g., *Borrelia afzelii, Borrelia burgdorferi, Borrelia garinii,* or *Borrelia mayonii).* In some embodiments, the compositions comprise one or more lipids, e.g., a phospholipid. In some embodiments, the lipids comprise one or more of phosphatidic acid (PA), phosphatidyl choline (PC), phosphatidylserine (PS), phosphatidylglycerol, phosphatidylethanolamine, and optionally one or more of at least one of ceramide, sphingomyelin, hexosyl cholesterol, acyl hexosyl cholesterol, or galactosyldiacylglycerol, and in some embodiments, the present technology is employed with alternative lipids, i.e., those that may be incorporated into a microorganism's membrane, such as sphingolipids. In some embodiments, the lipids are provided on a solid support. In some embodiments, the composition comprises a lipid panel, configured on a solid support, the lipid panel comprising phosphatidic acid (PA), phosphatidyl choline (PC), and/or phosphatidylserine (PS), and optionally one or more of at least one of ceramide, sphingomyelin, hexosyl cholesterol, acyl hexosyl cholesterol, galactosyldiacylglycerol, or phosphatidylethanolamine. In some embodiments, the solid support comprises a bead, plate, flow cell, flow chamber, microfluidic chamber, or microchip comprising a plurality of microfluidic chambers. In some embodiments, the composition is a reagent composition for use in a method described herein. In some embodiments, the composition is lyophilized.

### Methods

Disclosed herein are methods for the detection of infection with *Borrelia sp.* (e.g., *Borrelia afzelii, Borrelia burgdorferi, Borrelia garinii,* or *Borrelia mayonii).* In some embodiments, the methods comprise contacting an antibody-containing sample from a subject with a lipid, comprising one or more of phosphatidic acid (PA), phosphatidyl choline (PC), phosphatidylserine (PS), phosphatidylglycerol, phosphatidylethanolamine, and optionally one or more of at least one of ceramide, sphingomyelin, hexosyl cholesterol, acyl hexosyl cholesterol, or galactosyldiacylglycerol;, and in some embodiments, the present technology is employed with alternative lipids, i.e., those that may be incorporated into a microorganism's membrane, such as sphingolipids.

In some embodiments, the method includes allowing the binding of antibodies in the sample to the lipids; contacting the bound antibodies with a detectable binding agent; detecting the detectable binding agent; measuring the level of antibodies bound to each of the lipids; and comparing the measured levels of bound antibody to a value, such as a predetermined cutoff value or a value calculated based on the level of a maker in the sample measured in parallel or a control sample (e.g. a sample from a *Borrelia sp.* exposure naive subject).

The level of antibodies bound to the lipid can then be compared to a value to inform a diagnosis or treatment decision, such as, a diagnosis of Lyme disease or a *Borrelia* infection. The level of antibodies bound to the lipid can be used to monitor the progression of a treatment for Lyme disease or a *Borrelia* infection. The level of antibodies bound to the lipid, or after a series of measurements over a duration of weeks or months, can be used to estimate when the subject was exposed to *Borrelia sp.*

In some embodiments, if the measured level is greater than a predetermined antibody value for at least one of the lipids in the panel, the subject is diagnosed as positive for Lyme disease. In some embodiments, if the measured level is less than a predetermined antibody value for each of the lipids in the panel, the subject is diagnosed as negative for Lyme disease. In some embodiments, if the measured level is greater than a predetermined antibody value for at least one of the lipids in the panel, the subject is diagnosed as positive for Lyme disease and the method further comprises treating the subject for Lyme disease.

In some embodiments of any of the above-described methods, if the measured level is greater than a predetermined or control value for one or more of the lipids in the panel, the subject is diagnosed as positive for Lyme disease. In some embodiments, the method further comprises treating the subject for Lyme disease. In some embodiments, if the measured level is greater than a predetermined or control value for each of the lipids in the panel, the subject is diagnosed as positive for Lyme disease.

In some embodiments of any of the methods above, if the measured level is less than a predetermined, control, or calculated value for one or more of the lipids in the panel, the subject is diagnosed as negative for Lyme disease. In some embodiments of any of the methods above, if the measured level is less than a predetermined, control, or calculated value for each of the lipids in the panel, the subject is diagnosed as negative for Lyme disease.

As used herein the terms "predetermined value" and "control value" are used interchangeably. For example, in some embodiments, the predetermined value or control value is a cutoff value based on a naive control antibody level, either measured independently or measured using the same sample. A suitable cutoff value can be determined using routine techniques known to the skilled worker. In some embodiments, the cutoff value is calculated from a measured naive control level adjusted by a standard deviation or parameter based on the standard deviation (see e.g., Frey et al. [34]). For example, a cutoff value can be a mean naive control value + 2.291 standard deviations. In some embodiments, a subject is tested multiple times, and the control value (or predetermined value) comprises the test value of a previous sample from the same subject (*e*.*g*., to determine whether a subject is responding favorably to a treatment). In such an exemplary embodiment, the control value (the predetermined value) is greater than the test value if the subject is responsive to treatment, or the control value (the predetermined value) is equal to or less than the test value if the subject is non-responsive to treatment. While it is understood that in some situations a control value is not a predetermined value *per-se*, the terms "predetermined value" and "control value" are intended to encompass such situations. For example, if the control sample, (*e*.*g*., a known naive control sample, or a known positive sample) is tested simultaneously with the unknown, test sample, and the control value is determined at the time of test completion, then the "control value" is not necessarily "predetermined." However, it is known in advance of testing that the control sample will have a value indicative of a predetermined infection status (*e*.*g*., naive or positive).

In some embodiments, the method comprises (a) contacting an antibody-containing sample, derived from a subject, with a lipid comprising or consisting of phosphatidic acid (PA), phosphatidyl choline (PC), and/or phosphatidylserine (PS), and optionally one or more of at least one of ceramide, sphingomyelin, hexosyl cholesterol, acyl hexosyl cholesterol, galactosyldiacylglycerol, or phosphatidylethanolamine;; (b) incubating the sample with the lipids to allow binding of antibodies in the sample to the lipid(s) thereby forming an antibody-lipid complex; (c) detecting bound antibodies or antibody-lipid complexes, such as using a detectable binding agent; (d) measuring the level of antibodies bound to the lipid(s) based on the detecting step (c); (e) comparing the measured levels of step (e) to a value. In some further embodiments, the method comprises (a) contacting the antibody-containing sample with a reagent composition or lipid panel comprising the following lipids: phosphatidic acid (PA), phosphatidyl choline (PC), and/or phosphatidylserine (PS) ), and optionally one or more of at least one of ceramide, sphingomyelin, hexosyl cholesterol, acyl hexosyl cholesterol, galactosyldiacylglycerol, or phosphatidylethanolamine. In some embodiments, the method comprises, after the incubating step (b) and before the detecting step (c), a step of washing away unbound antibodies and/or another material or agent.

In some embodiments, the lipid is a phospholipid. In some embodiments, the phospholipid is phosphatidic acid, phosphatidyl choline, phosphatidylserine, phosphatidylglycerol, and/or phosphatidylethanolamine. In some embodiments one of ceramide, sphingomyelin, hexosyl cholesterol, acyl hexosyl or cholesterol, galactosyldiacylglycerol is additionally or alternatively detected. In some embodiments, the present technology is employed with additional or alternative lipids, i.e., those that are incorporated into a microorganism's membrane, such as sphingolipids.

In some embodiments, the lipid panel is configured on a solid support.

In some embodiments of any of the methods above, the detecting comprises detecting the bound antibodies or antibody-lipid complexes using a detectable binding agent.

In some embodiments of any of the methods above, the sample comprises or consists of a blood sample (e.g., whole blood), plasma sample, and/or serum sample.

In some embodiments of any of the methods above, the detecting step (c) comprises immobilizing antibody-lipid complexes to a solid support.

In some embodiments, the solid support comprises or consists of a plate, bead, flow cell, flow chamber, microfluidic chamber, and/or microchip comprising a plurality of microfluidic chambers.

In some embodiments, the detecting step (c) comprises flowing the reagent-composition through a flow-chamber. In some embodiments, the detection comprises a microfluidic chamber, microchip comprising a plurality of microfluidic chambers, or lateral flow assay.

In some embodiments, the subject is human and the detectable binding agent comprises an anti-human IgG comprising a detectable label or conjugated to a detectable agent. In some embodiments, the detectable agent comprises an enzyme, such as a horseradish peroxidase.

In some embodiments of any of the methods above, the subject previously tested positive for Lyme disease by standard two-tier (STT) testing.

In some embodiments of any of the methods above, the subject exhibits one or more of the following symptoms: erythema migrans, facial palsy, and arthritis; and/or the subject already tested positive for Lyme disease in standard two-tier test (STT). In some embodiments, the subject has exhibited the symptom for 3 months or less, 2 months or less, 1 month or less, 2 weeks or less, 1 week or less, 3 days or less, or 1 day or less.

In some embodiments of any of the methods above, the lipid is phosphatidic acid (PA) and the subject has exhibited the symptom for 2 weeks or less, 10 days or less, 1 week or less, 6 days or less, 5 days or less, 4 days or less, 3 days or less, 2 days or less, or 1 day or less.

In some embodiments of any of the methods above, the subject has not previously been treated for Lyme disease.

In some embodiments of any of the methods above, the subject is asymptomatic for Lyme disease, but may have come in contact with a blacklegged tick selected from *Ixodes scapularis*, *Ixodes pacificus*, *Ixodes ricinus*, and *Ixodes persulcatus.* In some embodiments, the possible contact between the subject and the tick was within 3 months, within 2 months, within 1 month, within 2 weeks, within 1 week, within 3 days, or within 1 day.

In some embodiments of any of the methods above, the subject is asymptomatic for Lyme disease and has previously been treated for Lyme disease. In some embodiments, the subject is asymptomatic for Lyme disease and has not previously been treated for Lyme disease.

In some embodiments of any of the methods above, the subject has previously been treated for Lyme disease and exhibits one or more of the following symptoms: erythema migrans, facial palsy, and arthritis; and/or the subject previously tested positive for Lyme disease in standard two-tier test (STT). In some embodiments, the subject has not received treatment for Lyme disease for at least 1 year, for at least 5 years, for at least 10 years, or for at least 15 years.

In some embodiments of any of the methods above, the method comprises after the comparing step, a step of treating the subject for Lyme disease.

In some embodiments of any of the methods above, the level of antibodies bound to the lipid(s) can be used to estimate when the subject was exposed to *Borrelia sp.* In some embodiments, the measured level of antibodies to the lipid(s) increasing over time is indicative of exposure to a *Borrelia sp.* within the last 30 days. In some embodiments, the measured level of antibodies to the lipid(s) decreasing over time is indicative of an exposure within 31 days or more. In some embodiments, the measured level of antibodies to phosphatidylserine (PA) decreasing over time is indicative of an exposure within 31-365, 31-200, or 31-99 days or more. In some embodiments, the measured level of antibodies to phosphatidyl choline (PC) decreasing over time is indicative of an exposure within 100-199 days. In some embodiments, the measured level of antibodies to phosphatidylserine (PS) decreasing over time is indicative of an exposure within 200-365 days.

In some embodiments of any of the methods above, the subject is being treated for Lyme disease. In some further embodiments, the method is used to monitor the progression of the treatment. In some further embodiments, the measured level of antibodies to phosphatidylserine (PS) is used to monitor the treatment wherein decreasing levels are indicative of positive treatment response.

In some embodiments of any of the methods above, the method can differentiate a subject infected with a *Borrelia* sp. (e.g., a subject having Lyme disease) from a subject infected with *T. pallidum* (e.g., having syphilis) but not a *Borrelia* sp.

### Kits

Disclosed herein are kits for the detection of infection with *Borrelia sp.* (e.g., *Borrelia afzelii, Borrelia burgdorferi, Borrelia garinii,* or *Borrelia mayonii).* In some embodiments, the kit comprises a composition described herein and one additional reagent or device. In some embodiments, kits include one or more lipids configured on a solid support or in a reagent composition, the lipids including one or more of phosphatidic acid (PA), phosphatidyl choline (PC), phosphatidylserine (PS), phosphatidylglycerol, phosphatidylethanolamine, and optionally one or more of at least one of ceramide, sphingomyelin, hexosyl cholesterol, acyl hexosyl cholesterol, or galactosyldiacylglycerol, and other lipids that may be incorporated into a *Borrelia sp.* membrane, such as a sphingolipid. In some embodiments, the additional reagent or device is a flow chamber, bead composition, ELISA reagent, anti-human IgG antibody, and/or a detectable reagent such as a HRP substrate. In some embodiments, the kit comprises instructions for using the components of the kit in the performance of a method described herein. In some embodiments, the kits comprise instructions, and are configured for in-home use.

### Illustrative Embodiments

Illustrative, non-limiting embodiments of the present technology are presented below.
1. A method comprising: (a) contacting an antibody-containing sample, derived from a subject, with a lipid panel configured on a solid support, the lipid panel comprising at least the following lipids: phosphatidic acid (PA), phosphatidyl choline (PC), and phosphatidylserine (PS), and optionally comprising one or more of ceramide, sphingomyelin, hexosyl cholesterol, acyl hexosyl cholesterol, galactosyldiacylglycerol, and phosphatidylethanolamine; (b) incubating the sample with the lipids to allow binding of antibodies in the sample to the lipids; (c) contacting the bound antibodies with a detectable binding agent; (d) detecting the detectable binding agent; (e) measuring the level of antibodies bound to each of the lipids based on (d); (f) comparing the measured levels of (e) to a predetermined value.
2. The method of embodiment 1, wherein the sample comprises a blood sample, a plasma sample, or a serum sample.
3. The method of embodiment 1, wherein the solid support comprises a bead, plate, test strip, or flow cell.
4. The method of embodiment 1, wherein the subject is human and the detectable binding agent comprises anti-human IgG comprising a detectable label.
5. The method of any one of embodiments 1-4, wherein the subject has been exposed to a Lyme disease pathogen for one week or less.
6. The method of any one of embodiments 1-4, wherein if the measured level is greater than the predetermined value for at least one of the lipids in the panel, the subject is diagnosed as positive for Lyme disease.
7. The method of embodiment 6, further comprising treating the subject for Lyme disease.
8. A method comprising: (a) contacting an antibody-containing sample, derived from a subject, with a reagent composition comprising the following lipids: phosphatidic acid (PA), phosphatidyl choline (PC), and phosphatidylserine (PS); the reagent composition optionally including one or more additional lipids selected from ceramide, sphingomyelin, hexosyl cholesterol, acyl hexosyl cholesterol, galactosyldiacylglycerol, and phosphatidylethanolamine; (b) incubating the sample with the reagent composition to allow binding of antibodies in the sample, if present, to the lipids thereby forming an antibody-lipid complex; (c) detecting bound antibodies or antibody-lipid complexes; (d) measuring the level of antibodies bound to PA, PC, and PS, and optionally the level of the one or more additional lipids based on (c); and (e) comparing the measured levels of (d) to a predetermined value for PA, PC, and PS and any of the additional lipids measured.
9. The method of embodiment 8, wherein the subject has been exposed to a Lyme disease pathogen for one week or less.
10. The method of any one of embodiments 8-9, wherein if the measured level is greater than the predetermined value for at least one of the lipids in the panel, the subject is diagnosed as positive for Lyme disease.
11. The method of embodiment 10, further comprising treating the subject for Lyme disease.
12. A composition comprising: phosphatidic acid (PA), phosphatidyl choline (PC), phosphatidylserine (PS); and at least one of a synthetic buffer, stabilizer, non-specific binding surface blocking agent, macromolecular crowding agent, antioxidant, preservative, and surfactant, wherein the PA, PC, and PS, are immobilized on a solid support, wherein the solid support comprises a plate, bead, flow chamber, microfluidic chamber, or microchip comprising a plurality of microfluidic chambers.
13. The composition of embodiment 12, comprised within a kit for detection of infection with *Borrelia sp.*
14. The composition of embodiment 13, wherein the *Borrelia* species is one of *B. afzelii* or *B. garinii.*
15. The composition of any one of embodiments 12-14, further comprising at least one of ceramide, sphingomyelin, hexosyl cholesterol, acyl hexosyl cholesterol, galactosyldiacylglycerol, or phosphatidylethanolamine, wherein the ceramide, sphingomyelin, hexosyl cholesterol, acyl hexosyl cholesterol, galactosyldiacylglycerol, or phosphatidylethanolamine immobilized on the solid support.
16. A method comprising: (a) contacting an antibody-containing sample, derived from a subject, with a lipid panel configured on a solid support, the lipid panel comprising at least the following lipids: phosphatidic acid (PA), phosphatidyl choline (PC), and phosphatidylserine (PS); (b) incubating the sample with the lipids to allow binding of antibodies in the sample to the lipids; (c) contacting the bound antibodies with a detectable binding agent; (d) detecting the detectable binding agent; (e) measuring the level of antibodies bound to each of the lipids based on (d); (f) comparing the measured levels of (e) to a predetermined value.
17. The method of embodiment 16, wherein the sample comprises a blood sample, a plasma sample, or a serum sample.
18. The method of embodiment 16 or 17, wherein the solid support comprises a bead, plate, test strip, or flow cell.
19. The method of any one of embodiments 16-18, wherein the subject is human and the detectable binding agent comprises anti-human IgG comprising a detectable label.
20. The method of any one of embodiments 16-19, wherein the subject exhibits one or more of the following symptoms: erythema migrans, facial palsy, and arthritis; and/or the subject tests positive for Lyme disease in standard two-tier test (STT).
21. The method of any one of embodiments 16-20, wherein the subject has exhibited the symptom for one month or less.
22. The method of any one of embodiments 16-21, wherein the subject is asymptomatic for Lyme disease, but may have come in contact with a blacklegged tick selected from *Ixodes scapularis* and *Ixodes pacificus*, and wherein the possible contact was within 1 month.
23. The method of any one of embodiments 16-22, wherein the lipid panel comprises one or more lipids selected from ceramide, sphingomyelin, hexosyl cholesterol, acyl hexosyl cholesterol, galactosyldiacylglycerol, and phosphatidylethanolamine.
24. The method of embodiment 1, wherein the subject has not previously been treated for Lyme disease.
25. The method of any one of embodiments 16-23, wherein the subject has been exposed to a Lyme disease pathogen for one week or less.
26. The method of any one of embodiments 16-24, wherein if the measured level is greater than the predetermined value for at least one of the lipids in the panel, the subject is diagnosed as positive for Lyme disease.
27. The method of embodiment 26, further comprising treating the subject for Lyme disease.
28. A method comprising: (a) contacting an antibody-containing sample, derived from a subject, with a reagent composition comprising the following lipids: phosphatidic acid (PA), phosphatidyl choline (PC), and phosphatidylserine (PS); (b) incubating the sample with the reagent composition to allow binding of antibodies in the sample, if present, to the lipids thereby forming an antibody-lipid complex; (c) detecting bound antibodies or antibody-lipid complexes; (d) measuring the level of antibodies bound to PA, PC, and PS based on (c); and (e) comparing the measured levels of (d) to a predetermined value for PA, PC, and PS.
29. The method of embodiment 28, wherein the subject has been exposed to a Lyme disease pathogen for one week or less.
30. The method of embodiment 28 or 29, wherein if the measured level is greater than the predetermined value for at least one of the lipids in the panel, the subject is diagnosed as positive for Lyme disease.
31. The method of embodiment 30, further comprising treating the subject for Lyme disease.
32. A composition, system or kit comprising: phosphatidic acid (PA), phosphatidyl choline (PC), phosphatidylserine (PS); and at least one of a synthetic buffer, stabilizer, non-specific binding surface blocking agent, macromolecular crowding agent, antioxidant, preservative, and surfactant, wherein the PA, PC, and PS are immobilized on a solid support, wherein the solid support comprises a plate, bead, flow chamber, microfluidic chamber, or microchip comprising a plurality of microfluidic chambers.
33. The composition system or kit of embodiment 32, comprised within a kit for detection of infection with *Borrelia sp.*
34. The composition, system or kit of embodiment 32 or 33, further including at least one of ceramide, sphingomyelin, hexosyl cholesterol, acyl hexosyl cholesterol, galactosyldiacylglycerol, or phosphatidylethanolamine, wherein the at least one of ceramide, sphingomyelin, hexosyl cholesterol, acyl hexosyl cholesterol, galactosyldiacylglycerol, or phosphatidylethanolamine, immobilized on the solid support.
35. The composition, system or kit of any of the previous embodiments wherein the *Borrelia* species is *β. burgdorferi or B. mayonii.*

### EXAMPLES

The following Examples are illustrative and are not intended to limit the scope of the claimed subject matter.

### Example 1: Antiphospholipid autoantibodies in Lyme disease arise after scavenging of host phospholipids by Borrelia burgdorferi.

With an average genome of approximately 1.3 mbp (1), many aspects of cellular metabolism are minimized or absent in *Borrelia burgdorferi.* Such an evolutionary genome reduction is possible because *B. burgdorferi* lives in close association with vertebrate and tick hosts (2), which are parasitized for many common metabolites synthesized de novo by other bacteria. Transport and scavenging mechanisms therefore replace many common synthetic pathways (3-5).

Characterized pathways of lipid metabolism are limited to the synthesis of phosphatidylcholine (PC), phosphatidylglycerol (PG), and 2 monogalactosyl lipids: galactosylcholesterol and galactosyldiacylglycerol (6). The acyl lipids derive from a common pathway in which glycerol-3-phosphate is twice acylated to form phosphatidic acid (PA), which is modified with choline, glycerol, or galactose (7, 8). Precursors for all membrane constituents are presumed to be scavenged from the host as *B. burgdorferi* lacks apparent synthetic pathways for fatty acids, cholesterol, and choline. Although the membrane of in vitro-cultured *B. burgdorferi* appears to be mostly composed of these 4 lipid species, chromatographic fractionation suggests a complex mixture of at least 11 components, most of which are unidentified (9).

The dependence on environmentally acquired fatty acids was determined with radiolabeling in *B. burgdorferi* (10) and the related *Borrelia hermsii* (11). In the former, the fatty acid composition of the membrane approximates that of the medium (12). Given this observation, and that the otherwise well-conserved *fad* system of fatty acid translocation is also absent from *Borrelia* (1), uptake into the periplasm is likely to be passive via diffusion across the membranes. This phenomenon is likely facilitated in spirochetes by their lack of an exopolysaccharide coat, which leaves them unusually accessible to hydrophobic moieties. Similarly, cholesterol acquisition has been shown to be mediated by the fusion of host-derived membrane vesicles with *B. burgdorferi* (13).

Although the majority of characterized antibodies against *B. burgdorferi* recognize protein antigens, anti-lipid antibodies also develop during infection. Antibodies against both galactosylcholesterol and galactosyldiacylglycerol are raised in Lyme disease and remain elevated after treatment (14). These antibodies have been shown to cross-react with mammalian gangliosides (15), and this reaction has been suggested as a possible cause of neurologic symptoms of borreliosis. Lipids also stimulate the innate immune response, with natural killer T (NKT) cells being activated by the galactosyldiacylglycerol (16) of *B. burgdorferi* to produce immunostimulatory IFN-γ, which is responsible for the recruitment of macrophages and, ultimately, the clearing of infection (17, 18).

Although not widely studied, anti-lipid antibodies are described in other infectious diseases (19-21) as well as in systemic autoimmune conditions (22, 23). Syphilis, caused by the related spirochete *Treponema pallidum*, is diagnosed by serology to detect antibodies against either treponemal (lipoprotein) or nontreponemal (lipid) epitopes. The nontreponemal rapid plasma regain (RPR) or venereal disease research laboratory (VDRL) tests use an antigen mixture of cardiolipin (CL), lecithin, and cholesterol, of which CL is thought to be the most seroreactive (24). CL is found in both the mitochondrial membranes of the host and the membranes of infecting treponemes (25). The high titers seen during an active infection are thought to result from CL and other lipids liberated from damaged host cells as well as those presented directly by the bacteria (26). Quantitative measurement of the nontreponemal antibodies, which decline within 6 to 12 months of bacterial clearance, allows for the monitoring of treatment success (27). This quantitation informs clinical decisions regarding the need for further treatment. In the absence of an equivalent test for Lyme disease, approaches to the management (and even the definition) of persistent symptoms vary widely (28).

Serology is the mainstay in the clinical diagnosis of Lyme disease (29). CDC-recommended standard testing algorithms use a 2-stage protocol of an ELISA followed by either Western blotting or a second ELISA (30). Current tests are accurate in the detection of established infections, but during the earliest stages of infection, most antibody tests are only 50%-60% sensitive (31). Additionally, these antibody tests can remain positive for decades after antibiotic treatment (32), limiting their usefulness in determining treatment success or identifying reinfection. To identify antiphospholipid antibodies with potential for use as diagnostic markers of Lyme disease, the incorporation of diverse phospholipids by *B. burgdorferi* was investigated. This was followed with a survey of antiphospholipid antibodies in *B. burgdorferi*-infected mice and the identification of an antibody panel able to discriminate between healthy controls and patients with Lyme disease. Unlike current tests, the antibodies identified also allowed for monitoring of the response to therapy, with titers declining in the year following treatment.

### Results

### B. burgdorferi is a lipid auxotroph.

*B. burgdorferi* is unable to grow in a lipid-depleted growth medium (delipidated BSK [dBSK]), with growth being restored by supplementation with fatty acids and cholesterol (Figure 8A). A standard Barbour-Stoenner-Kelly II (BSK) growth medium was stripped of lipids by organic extraction, yielding a medium unable to support the growth of *B. burgdorferi.* On the basis of previous analyses of borrelial membranes (6, 9), cholesterol and fatty acids were supplied at a molar ratio of 1:4, with the fatty acids consisting of an equal mix of palmitic (16:0) and oleic (18:1) acid. Upon supplementation with lipids, the growth was restored in a concentration-dependent manner. Darkfield microscopy confirmed that the cells cultured in lipid-supplemented dBSK had a morphology and motility similar to those of cells cultured in standard BSK.

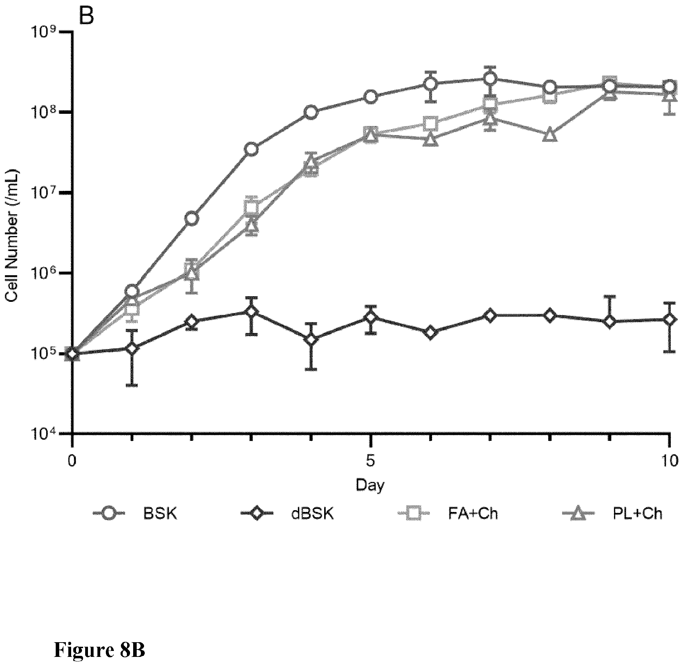

In addition to fatty acids, intact phospholipids can be used as a lipid source for growth (Figure 8B). A similar growth curve was seen with fatty acids (200 µM palmitic acid, 200 µM oleic acid) and an equivalent concentration of phospholipids (100 µM PG, 100 µM PC). Cholesterol was included at 100 µM in all cultures.

### B. burgdorferi accumulates environmental phospholipids.

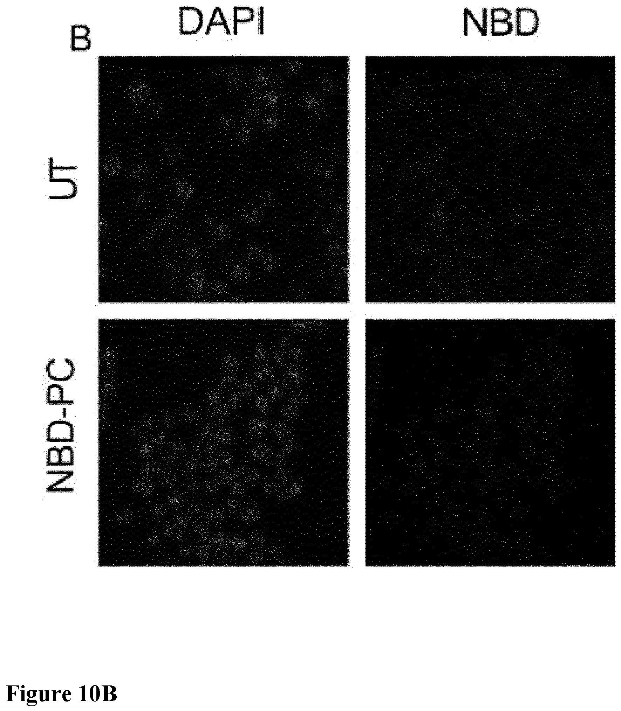

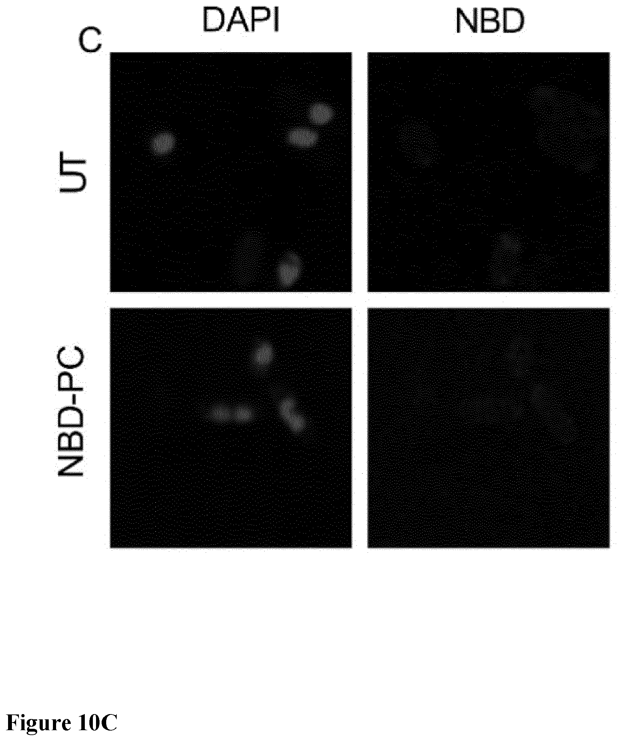

Given the utilization of phospholipids from the growth medium, their uptake was assayed using fluorescent (nitrobenzoxadiazole-labeled [NBD-labeled]) phospholipid analogs. Although *B. burgdorferi* can synthesize PC and PG from precursors (7), these previously identified *B. burgdorferi* phospholipids can also be acquired intact from the growth medium (9). Further, 3 phospholipids that, to our knowledge, have not previously been identified in *B. burgdorferi* (phosphatidylethanolamine [PE], phosphatidylserine [PS], and PA) were also incorporated into borrelial membranes when present in the medium (Figure 9). Under the same conditions, NBD-PC did not enter the membranes of either *E. coli* or *S. aureus* (Figure 10), suggesting that this phenomenon may be particular to *Borrelia.*

Incorporation of all phospholipids occurred within 1 hour and was largely constant over 6 hours, indicative of a rapidly established equilibrium between cell membranes and the medium. The disparity in intensity between the 5 fluorophores may indicate that the borrelial membrane was differently accessible to the various lipid species, possibly as a function of the lipoprotein coat. However, the solution dynamics of the assayed phospholipids is also likely to affect their accessibility. Their solubility and critical micelle concentrations in the dBSK medium were not determined but are likely to be variable across the group.

Using a competition assay between phospholipids with 2 different fluorophores (NBD-PC and Texas Red-PE), we show that phospholipids entered the membrane at approximately the same relative concentration as in the medium (Figure 11). This is indicative of nonspecific uptake and is consistent with a model of random diffusion into the membrane. Fluorescence was detectable for up to 4 days after the initial labeling, with a half-life of approximately 1 day (Figure 12). Although the decline in signal could be a result of either degradation or membrane turnover (as fluorophores are shed to the medium), the *B. burgdorferi* genome predicts neither phospholipases nor fatty acid catabolic pathways, suggesting that degradation was unlikely.

Antiphospholipid antibodies are raised during murine infection.

The seroreactivity of these noncanonical membrane components was assessed by ELISA using a mouse model of *B. burgdorferi* infection. Infected (4 weeks) and naive mouse sera were probed for antibodies against the phospholipids PG, PC, PA, PS, PE, and the PG dimer CL. Galactosylcholesterol antibodies, which have been extensively studied in Lyme disease (14, 33), were also assayed as a reference. Mice were infected for 28 days before the collection of serum, at which point they typically manifest active joint disease as well as IgG and IgM antibody responses (34).

After 4 weeks of infection, antibodies were raised against 5 of the 6 assayed phospholipids, as well as galactosylcholesterol (Figure 13A). The antibody responses varied in magnitude, with post-infection titers of anti-PC and anti-PA being the greatest. Anti-PC and anti-PA also showed the highest relative increases from the naive levels at approximately 30- and approximately 13-fold, respectively. Although the final titer of anti-PE was modest (reaching about the same level as anti-PA and anti-PG in the naive mice), this represented a 10-fold increase from the lowest basal titer of the lipids assayed. Increases in anti-PS (~4-fold) and anti-PG (~3-fold) were smaller, but for every antibody except anti-CL, the comparison of naive and infected samples was statistically significant (P < 0.05).

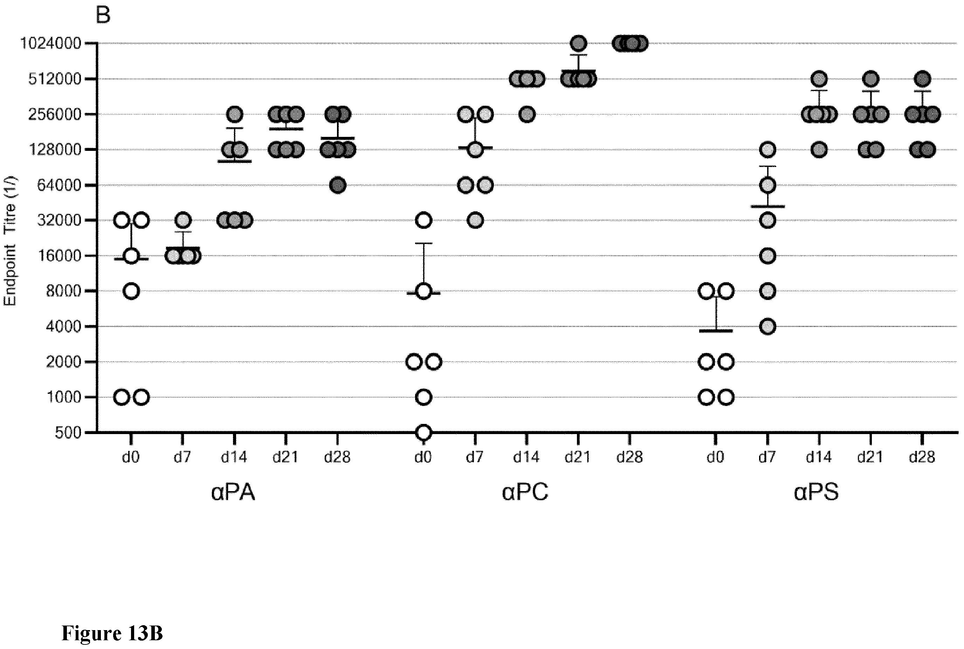

To investigate the kinetics of the antiphospholipid responses, 6 additional mice were serially sampled over the first 4 weeks of infection (Figure 13B). Three antibodies (anti-PA, anti-PC, and anti-PS) were measured at each of 5 time points, with all 3 showing a similar pattern of induction. Titers begin to increase by day 7 and increased most dramatically between days 7 and 14. For 2 of the antibodies, the endpoint titers were stable over the later time points, suggesting that a peak was reached within 14 days (for anti-PS) or 21 days (anti-PA). Anti-PC continued to rise out to day 28 and may have been found to reach higher titers with prolonged infection.

### Antiphospholipid antibodies in human serum.

Human serum samples were probed for IgG antibodies against 4 of the phospholipids assayed above: PA, PC, PS, and CL. In order to mimic a higher-throughput diagnostic assay of the kind used in clinical settings, these immunoassays were performed at a single dilution, with the OD normalized to a panel of 12 uninfected controls run in parallel. Naive, untreated, and post-treatment groups each comprised 12 individuals (Tables 1-3). An additional group consisting of sera from 12 patients with syphilis (RPR- and treponemal IgG-positive) was used to assess the cross-reaction of the antiphospholipid tests. Under the assay conditions tested, no significant difference was found between the syphilis and control sera (Figure 14).

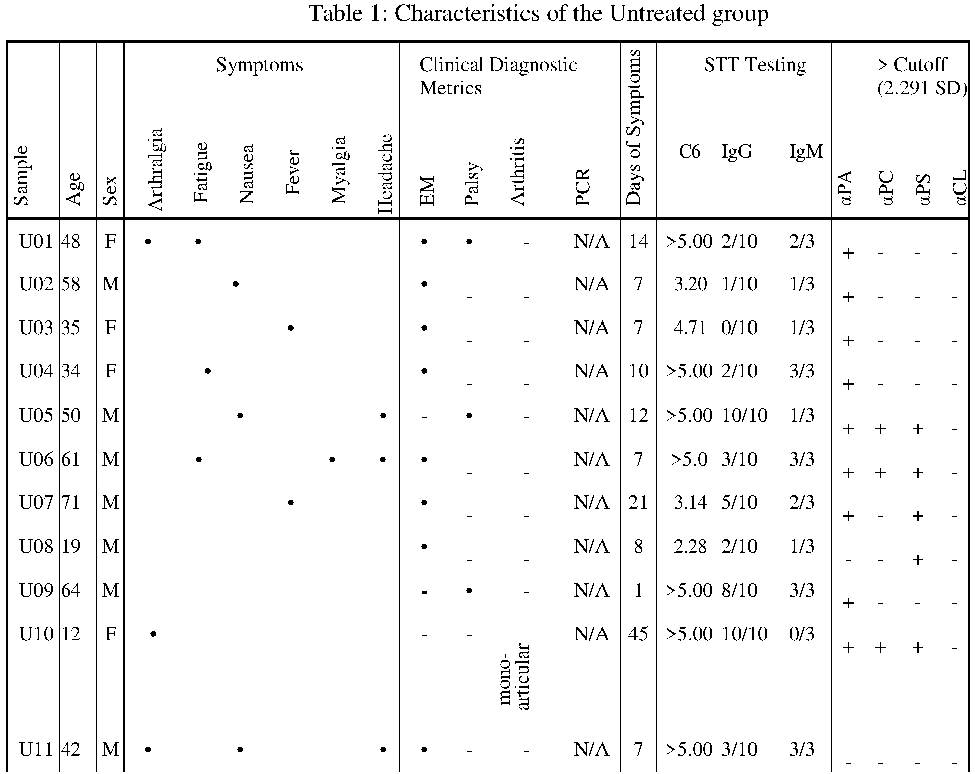

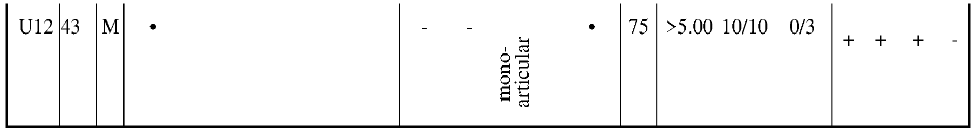

In table 1, for a positive result by standardized two-tier (STT) testing, a positive (≥1.1) or equivocal C6 screening ELISA (≥0.9) and either ≥5/10 IgG or ≥2/3 IgM bands by Western blot are required. Where STT testing is negative, clinical diagnoses are also made in the presence of characteristic symptoms such as the erythema migrans or Bell's palsy. PCR from the synovial fluid is rarely performed but can also confirm a *B. burgdorferi* infection. For antiphospholipid ELISAs, the cutoff for a positive result was the mean of 12 naive controls + 2.291 standard deviations. One sample (U11) was negative for all phospholipids assayed. Mean age 44.8 years, median 45.5.

Sera from 12 untreated individuals were collected at the time of diagnosis, 1 to 75 days after the onset of symptoms (mean = 17.8 days, median = 9.0 days). This time span includes the early stages of infection, when existing diagnostic antibodies have low sensitivity (31): 5 samples were collected 0-7 days after diagnosis, 4 samples 8-14 days after diagnosis, and 3 samples more than 21 days after diagnosis. The titers of 3 of the 4 antibodies assayed were significantly different (*P <* 0.05) in the control and untreated sera (Figure 15). As in the mouse model, anti-CL titers were unchanged during human infection. Treated sera were collected from 12 individuals at least 1 year (1-13 years, mean = 6 years, median = 5 years) from the initial diagnosis and treatment of Lyme disease, with all samples remaining positive by standardized two-tier (STT) testing.

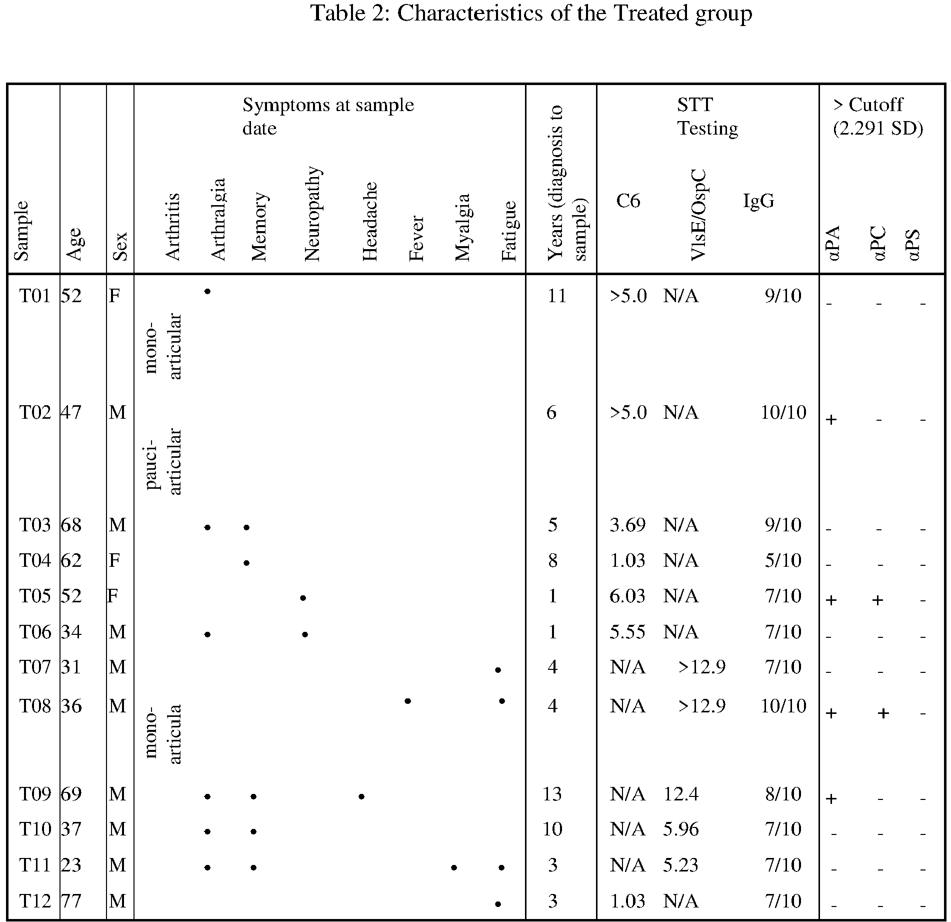

In table 2, STT testing was performed as above, but IgM data are discounted where symptoms have persisted for >30 days. During the sample collection period, two different screening ELISAs were used at the collection site. Both the C6 ELISA and the VlsE/OspC combined immunoassay (DiaSorin) are equivocal at an index >0.9 and positive at >1.1. Mean age 49.0 years, median 49.5.

The cutoff value, which defined a positive sample, was established using a panel of 12 negative control samples from healthy donors. The 3 phospholipids that were elevated during infection were assessed for diagnostic value using a cutoff value equal to the mean of the naive controls plus 2.291 SDs. This was derived using the method described by Frey et al. (35). In the untreated group, antiphospholipid titers predicted infection in 10 of 12, 4 of 12, and 6 of 12 samples for anti-PA, anti-PC, and anti-PS, respectively. If the 3 tests are combined as a panel and elevation of any single antibody constitutes a positive result, the diagnostic efficiency increased to 11 of 12. By the same definition, only 4 of the post-treatment samples were positive: 2 were elevated for anti-PC and anti-PA and 2 for anti-PA alone. Two of the patients with syphilis (16.7%) passed the cutoff for anti-PA only; it may be notable that these 2 patients also had the lowest treponemal IgG titers (Table 3).

**Table 3: Characteristics of the Syphilis group**

| Sample | Age | Sex | Syphilis IgG | | RPR test | aPL results | | |
|---|---|---|---|---|---|---|---|---|
| | | | Result | Interpretation | Result | αPA | αPC | αPS |
| S01 | 58 | M | 2.1 | positive | reactive | + | - | - |
| S02 | 50 | M | > 8.0 | positive | reactive | - | - | - |
| S03 | 44 | M | > 8.0 | positive | reactive | - | - | - |
| S04 | 40 | F | > 8.0 | positive | reactive | - | - | - |
| S05 | 40 | M | > 8.0 | positive | reactive | - | - | - |
| S06 | 49 | F | > 8.0 | positive | reactive | - | - | - |
| S07 | 46 | F | > 8.0 | positive | reactive | - | - | - |
| S08 | 28 | F | > 8.0 | positive | reactive | - | - | - |
| S09 | 43 | M | > 8.0 | positive | reactive | - | - | - |
| S10 | 45 | F | > 8.0 | positive | reactive | - | - | - |
| S11 | 49 | M | > 8.0 | positive | reactive | - | - | - |
| S12 | 50 | M | 1.8 | positive | reactive | + | - | - |

In table 3, syphilis was diagnosed by a treponemal test for protein antigens and a nontreponemal test for lipid antigens. The treponemal IgG test (Bioplex 2200) is positive at an index >1. The nontreponemal test used at this collection site (ASI card test) gives only a qualitative reactive/unreactive result. For antiphospholipid ELISAs performed here, the cutoff for a positive result was the mean of 12 naive controls + 2.291 standard deviations. Syphilis sera were purchased from Precision Biospecimens.

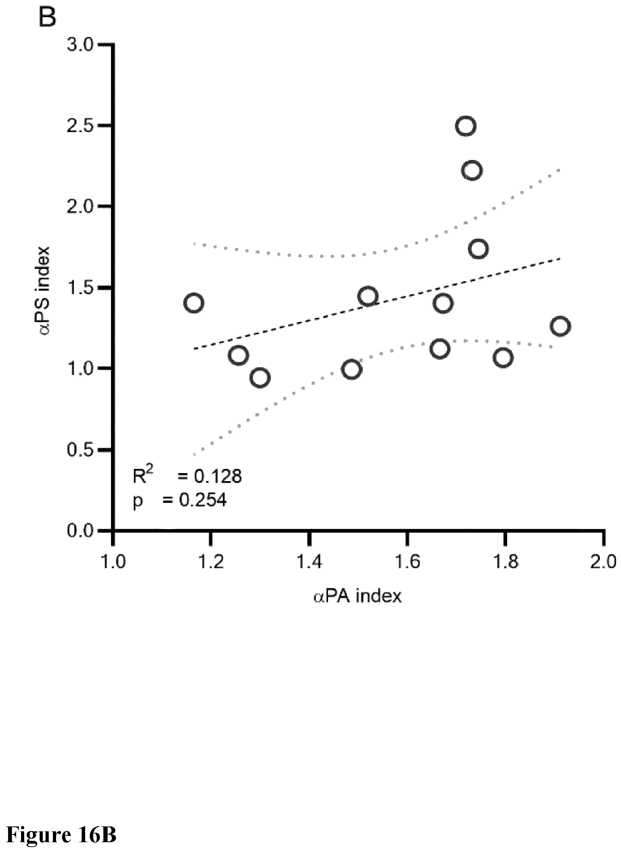

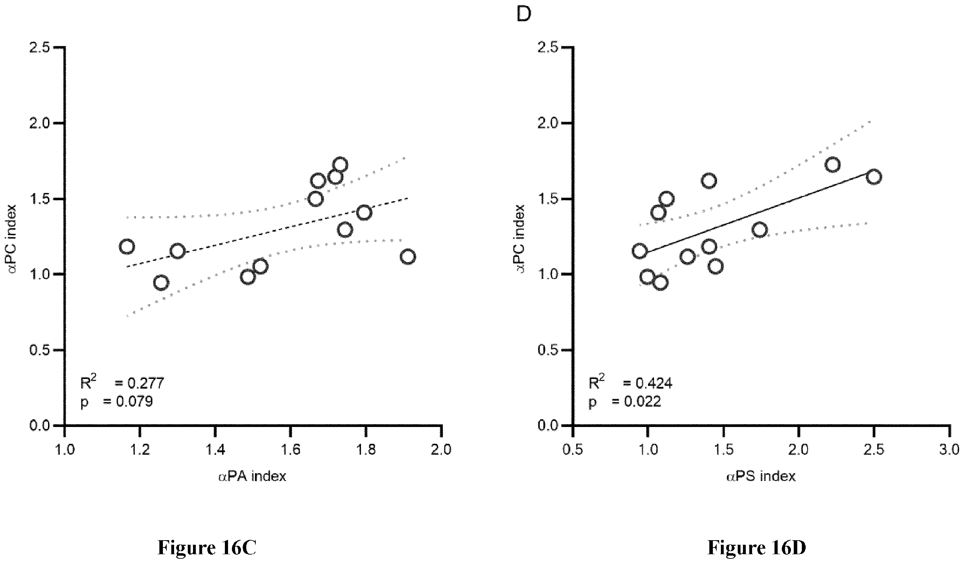

To assess whether the 3 antibodies arise independently, the pairwise correlations were analyzed by linear regression (Figure 16) and found that only anti-PC and anti-PS were significantly correlated. Further, each individual was ranked from 1 to 12 by their responses to each of the phospholipids. While some patients were consistently high or low across the 3 antibodies assayed, others showed variable responses to each.

In addition to the treated group described above, the kinetics of antibody responses to infection and treatment were studied using serial samples collected before and at various time points after treatment. Ten patients were evaluated, with 2, 3, or 4 samples collected for each patient. Five patients had pre-therapy (day 0) samples and all patients had convalescent samples, collected a median of 31 days (range, 23-49 days) from the start of therapy and 46.5 days (range, 30-62 days) from the start of illness. Follow-up samples were collected 4-12 months after the start of antibiotic treatment (Table 4). To correct for variation in the absolute titers between patients, the data are presented as a percentage of the maximum titer for each antibody in each patient (Figure 17). Titers of all 3 antibodies peaked within 30 days of treatment and began to decline by day 100 after the beginning of treatment. Anti-PA levels declined the least (to 79% of peak titer at 200-365 days) and the slowest, with a slope of 0.05. More pronounced declines were observed for anti-PC (to 74%, slope = 0.08) and anti-PS (to 69%, slope = 0.09). Anti-PA and anti-PC were on average higher on day 0, suggesting that these antibodies may arise faster than anti-PS.

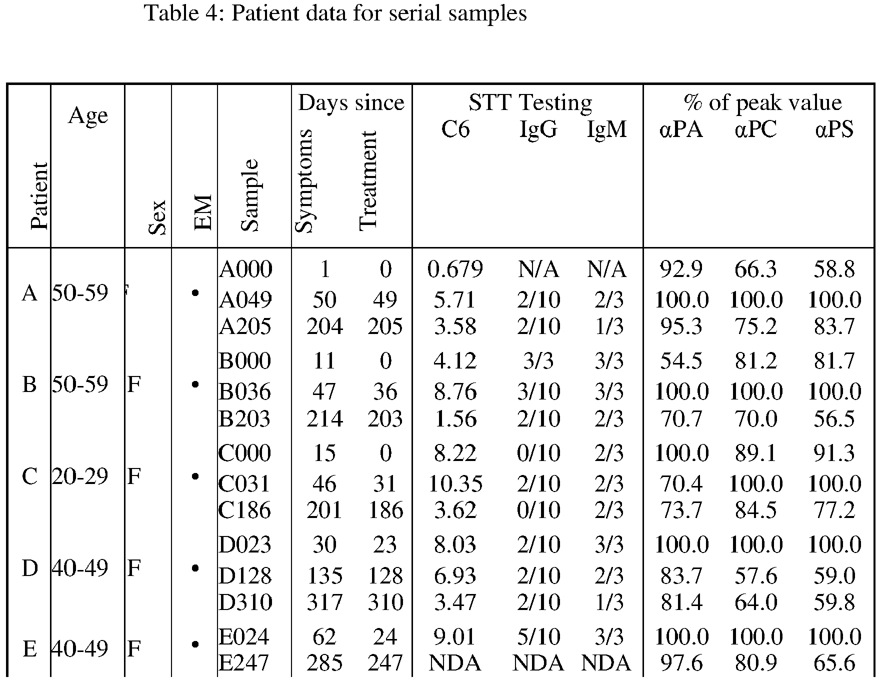

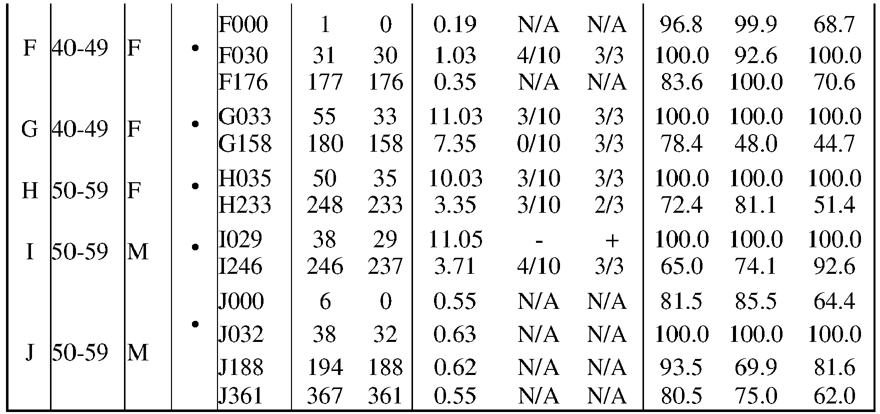

In table 4, data from 10 patients diagnosed with Lyme disease by the presence of an erythema migrans rash (EM). Multiple (2-4) samples were taken from each patient at different time points up to 1 year after the beginning of treatment. D0 (pre-treatment) samples were available for 5 patients. Antiphospholipid titers are shown as a percentage of each individual's peak value as measured by optical density at a single dilution. NDA = no data available. N/A indicates testing was not performed; where the C6 ELISA is negative second tier testing is not carried out. For one sample, only the interpretation (+/-) and not the full Western blot data were available.

### Discussion

*Borrelia* are fastidious bacteria requiring a complex culture medium with multiple undefined components for in vitro growth (36, 37). As a result, studies of nutrition and metabolism are difficult and rely on modified media depleted of specific nutrients (38, 39). Here, we developed an organic delipidation process for the complex components of the culture medium (serum, BSA, and yeast extract) and demonstrate that *B. burgdorferi* was unable to grow in the delipidated media. Using these media, the lipid content of the medium could be defined through the addition of purified lipids in order to identify the lipids that could support *B. burgdorferi* growth. Although the necessity of an external lipid source was predicted by a lack of synthetic enzymes in the *B. burgdorferi* genome (1), auxotrophy has not, to our knowledge, previously been demonstrated experimentally. The accessibility of the borrelial membrane to fatty acids was previously demonstrated by incorporation of radiolabeled palmitic and oleic acids; the fatty acid composition of cells was shown to mirror that of their growth medium (12).

The utilization of host-derived fatty acids is a common metabolic shortcut in host-associated growth; even organisms possessing pathways for de novo synthesis can bypass them in the presence of an alternative source (40). In addition to facilitating evolutionary genome reduction, the utilization of host-derived fatty acids may serve to suppress the immune responses of NKT cells. CD1d-mediated activation of NKT cells is dependent on both the polar head and fatty acid tail groups of lipids, with small variations in fatty acid composition profoundly affecting the cellular response (41, 42). The ligand-binding site of CD1d has a particular affinity for acyl chains containing characteristic microbial signatures such as anteiso methyl branches and shorter chain lengths; incorporation of host fatty acids into PG and PC can render them up to 100-fold less immunostimulatory than microbe-derived equivalents (43).

Fatty acids appear to be acquired by diffusion into the *0* membrane (44). The ready accumulation of fatty acids and cholesterol (13) suggests the possibility that other exogenous lipid species may also enter borrelial membranes. This was confirmed here by incorporation of fluorescently labeled phospholipid conjugates into *B. burgdorferi* cells. The accumulation and utilization of intact PG and PC molecules presents an alternative pathway for membrane metabolism in environments where fatty acids used for phospholipid synthesis are scarce. In BSK medium, the lipid source is rabbit serum, but in vivo *B. burgdorferi* is only transiently found in the bloodstream (45, 46), and so this medium is unlikely to model nutrient availability for the in vivo growth of the pathogen. The plasma concentration in humans of free fatty acids is approximately 200 µM (47), a concentration likely to be sufficient for growth of *B. burgdorferi.* In other tissues, however, the fatty acid concentration varies widely (48) and can be 10- or 100-fold lower than that of phospholipids (49). Phospholipids may therefore be the most significant lipid source for *B. burgdorferi* during growth in the vertebrate host.

In addition to the canonical *B. burgdorferi* membrane phospholipids PG and PC, other (non-borrelial) phospholipids are also able to access the membrane and accumulate in cells. PE, PS, and PA from the medium entered the membrane within 1 hour. PA, PE, and PS are all components of mammalian cell membranes and likely to be encountered by *B. burgdorferi* through the course of infection. PA is also likely to be present in *Borrelia* as an intermediate in the synthesis of PG and PC (7), although it is not detected in BSK-cultured cells (6, 9), suggesting this intermediate is short-lived and that significant accumulation requires an external source of PA. In serum (and therefore the BSK medium), the principal phospholipid is PC (at ~2000 µM), but PE is also abundant (435 µM). Other phospholipids are present only at low concentrations (<10 µM; ref. 47). In other tissues, however, the relative concentrations of lipid species varies (49), which may affect the repertoire of lipids available to infecting cells at different sites of infection.

The use of host lipids may be a general property of spirochetes. The membranes of in vivo-cultivated *Treponema pallidum* (12) are significantly more diverse than those of in vitro-cultured *Treponema denticola* (50), and include phospholipids for which treponemes have no characterized synthetic pathways. In vivo, the fusion of membrane vesicles derived from host cells is thought to be the mechanism by which cholesterol is acquired by *B. burgdorferi* (13). Given their origin, such vesicles are likely to contain a great diversity of lipid species, any of which might incorporate into the borrelial membrane with possible consequences for host immune responses. While only 5 phospholipids were assayed here, it is likely that other lipid classes (e.g., sphingolipids and sterols) will also integrate into the membrane and that - to some extent - the lipid composition of *B. burgdorferi* will approximate that of the environment.

Antibodies against the borrelial membrane constituent galactosylcholesterol are well characterized in Lyme disease (14, 33). Given this, and the utility of phospholipids in the diagnosis of the related *T. pallidum*, a screen of phospholipid antibodies was conducted in naive and *B. burgdorferi*-infected mice. Antibody responses to phospholipids, including those not synthesized by *B. burgdorferi* itself, were raised as early as day 7, with the greatest increases seen within 14 days. During infection, IgG antibodies against all assayed phospholipids were raised, with the exception of CL. Antibodies against galactosylcholesterol, which have been characterized in humans but not previously in mice, were also elevated.

Three of the elevated antibodies in mice were tested for potential diagnostic value in human samples. We chose anti-PA and anti-PC, as they reached the highest titers and had the greatest increase (versus the naive controls) of those assayed in mice. Anti-PS was included as it entered *B. burgdorferi* cells most readily, after PC. On the basis of the results from the mouse studies, we also included anti-CL as a potential negative control. Anti-PA, anti-PC, and anti-PS were all elevated in infection. Interestingly, the antibody titers against different phospholipids did not correlate, which indicated a degree of independence in the responses. It is unclear whether this reflects localization of the organism to specific tissues, in which *B. burgdorferi* may have scavenged different phospholipids, as our sample size was too small to separate patients by manifestation. However, this independence may be valuable for the development of diagnostics if the different antibodies correlate with different clinical characteristics.

As expected, no significant response to CL was observed in the patients with Lyme disease. IgG anti-CL has been previously described in a minority of patients with Lyme disease (51). However, these antibodies were most common in those with neurological symptoms (52), which were described in 3 of the 12 individuals in our untreated group and 2 of the 12 individuals in our treated group. Lyme disease sera are not thought to cross-react with the CL-based nontreponemal tests for syphilis (34, 53). Two positive tests for anti-CL were seen in patients long after treatment, but the significance of this is unclear. A group of 12 patients with syphilis were also tested for anti-PA, anti-PC, and anti-PS antibodies. No significant difference between control and syphilis sera was found for any of the 3 antibodies.

Although all the phospholipids assayed may be present (constitutively or conditionally) in the borrelial membrane, the source of the antigens that precipitate antibody production was undetermined here. The antibodies may be part of a specific adaptive response: other borrelial lipid antigens are presented to T cells by CD1d after phagocytosis (54, 55), and a humoral response to lipid antigens has been demonstrated after infection by other pathogens (26, 56). Alternatively, phospholipid antigens may arise indirectly after host tissue damage during the course of infection (57). In *T. pallidum* infection (26), both mechanisms contribute. *B. burgdorferi* does not cause tissue destructive lesions comparable to the chancres that develop with syphilis infection, which may account for the lack of an anti-CL response. CL is found predominantly in the mitochondria, and, as such, *B. burgdorferi* may not encounter it in significant quantities in the absence of cellular destruction.

Although coinfection of syphilis or relapsing fever spirochetes (such as *Borrelia hermsii*) with *B. burgdorferi* is unlikely, one limitation of this study is that we did not have access to sufficient sample volumes to test for these or other spirochetal diseases. *Borrelia miyamotoi* coinfections can occur alongside *B. burgdorferi* but are currently much less common in the northeastern United States, where our samples were collected. Surveillance of infection rates in ticks in the Northeast typically show 10- to 50-fold less infection with *B. miyamotoi* than with *B. burgdorferi* (58). *B. miyamotoi* infection is treated with the same antibiotics as for Lyme disease, and so possible coinfections would probably also have resolved in our treated group. All patients had Lyme disease, as confirmed by either erythema migrans (EM) rash or STT testing, but we cannot rule out possible coinfections with *B. miyamotoi.*

Antibodies against PA and PS were the most reliable diagnostic indicators, identifying 10 of 12 infections and 6 of 12 infections in the untreated group, respectively. Anti-PC was less sensitive, and only 4 individuals in the untreated group passed the positivity cutoff (Table 5). When the antibodies are taken together as a panel, elevation in any 1 of the 3 antibodies diagnosed 11 of the 12 untreated samples. Three of those identified by antiphospholipid antibodies tested negative by conventional STT testing, suggesting that these antibodies may enhance diagnostic coverage early in infection, when the STT test has low sensitivity (59). Further studies with greater numbers of samples will be needed to test this possibility. It is possible that antibodies against phospholipids may arise earlier because of the T cell-independent pathway by which these molecules are processed and activate the immune system. Invariant NKT (iNKT) cells stimulated with lipid antigens interact directly with B cells and may provoke faster activation than peptide-specific T cell interactions do (60, 61).

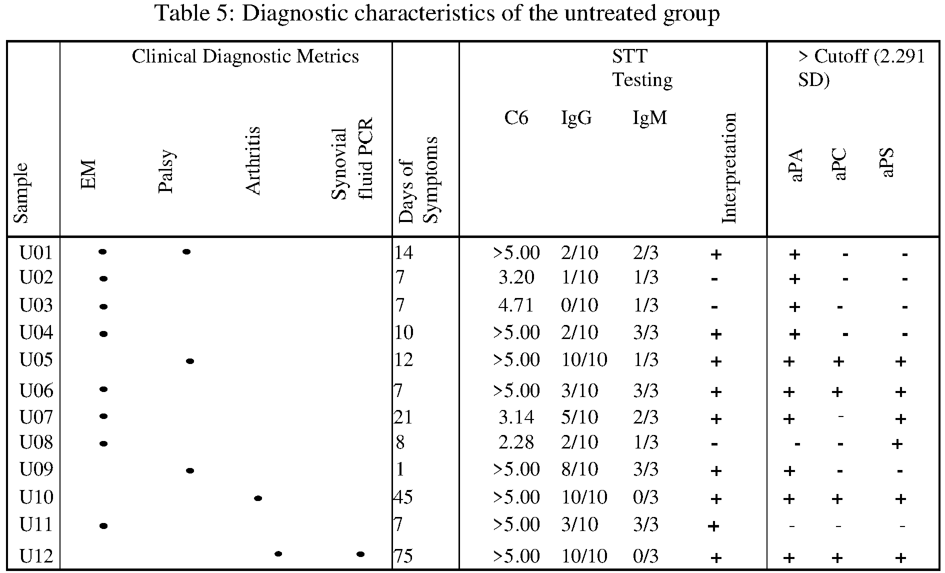

In table 5, for a positive result by standardized two-tier (STT) testing, a positive (≥1.1) or equivocal C6 screening ELISA (≥0.9) and either ≥5/10 IgG or ≥2/3 IgM bands by Western blot are required. Where STT testing is negative, clinical diagnoses are also made in the presence of characteristic symptoms such as the erythema migrans or Bell's palsy. PCR from the synovial fluid is rarely performed but can also confirm a B. burgdorferi infection. For antiphospholipid ELISAs, the cutoff for a positive result was the mean of 12 naive controls + 2.291 standard deviations. One sample (U11) was negative for all phospholipids assayed.

Two of the 3 antibodies that were elevated prior to treatment declined significantly in the post-treatment samples, and using the same diagnostic cutoff, only 4 of the 12 samples from the post-treatment group were positive for anti-PL antibodies (Table 6). In the same group, all 12 individuals remained positive by STT testing, despite no evidence of new infection. Again, the difference in the processing of phospholipids and the generation of antibodies has been suggested as the reason why antiphospholipid antibodies may be short-lived in the absence of continued stimulation. Although cognate iNKT help of B cells generates a robust initial response, it may not result in the generation of memory B cells (62). In 2 other long-term infections, *Mycobacterium tuberculosis* (19) and *Helicobacter pylori* (63), antiphospholipid antibodies are found to decline after successful treatment. A limitation of these post-treatment samples is that they were collected from patients under evaluation for nonspecific symptoms long after their initial diagnosis and treatment; other conditions unrelated to Lyme disease may have been present. Although none had clinical signs of reinfection, the lack of a gold-standard test for the eradication of *B. burgdorferi* makes it impossible to be certain that the treated patients were free of infection at the time of sampling. These factors combined may account for the 4 positive results in the treated group.

**Table 6: Patient characteristics of the treated group**

| Sample | Years between diagnosis and sample | STT Testing | | | | > Cutoff (2.29 SD) | | |
|---|---|---|---|---|---|---|---|---|
| | | C6 | VIsE/OspC | IgG | Result | αPA | αPC | αPS |
| T01 | 11 | >5.0 | N/A | 9/10 | + | - | - | - |
| T02 | 6 | >5.0 | N/A | 10/10 | + | + | - | - |
| T03 | 5 | 3.69 | N/A | 9/10 | + | - | - | - |
| T04 | 8 | 1.03 | N/A | 5/10 | + | - | - | - |
| T05 | 1 | 6.03 | N/A | 7/10 | + | + | + | - |
| T06 | 1 | N/A | 5.55 | 7/10 | + | - | - | - |
| T07 | 4 | N/A | >12.9 | 7/10 | + | - | - | - |
| T08 | 4 | N/A | >12.9 | 10/10 | + | + | + | - |
| T09 | 13 | N/A | 12.4 | 8/10 | + | + | - | - |
| T10 | 10 | N/A | 5.96 | 7/10 | + | - | - | - |
| T11 | 3 | N/A | 5.23 | 7/10 | + | - | - | - |
| T12 | 3 | 1.03 | N/A | 7/10 | + | - | - | - |

In table 6, all members of the treated group had a historical diagnosis of Lyme disease and continued to test positive by STT testing. For a positive result by standardized two-tier (STT) testing, a positive or equivocal screening immunoassay (index ≥ 0.9) and ≥ 5/10 IgG bands by Western blot are required. IgM testing is not applied for infections of longer than 30 days. During the sample collection period, two different screening ELISAs were used at the collection site. Both the C6 ELISA and the VlsE/OspC combined immunoassay (DiaSorin) are equivocal at an index > 0.9 and positive at > 1.1.

To better assess the evolution in antiphospholipid titers during treatment, serial samples were analyzed in a group of 10 patients during treatment and recovery. Although true baseline (pre-infection) titers were not available for these patients, a marked decline was observed in antibody titers over the period of 1 year. This decline was more pronounced for anti-PC and anti-PS titers, suggesting that these antibodies may be of greatest value in monitoring the response to treatment - similar to the use of the nontreponemal tests used to track responses to therapy in syphilis.

Two major gaps in Lyme disease diagnostics are the inability to identify whether continued infection may play a role in post-therapy symptoms and the inability to identify patients with repeat infection. A return to baseline of antiphospholipid antibody levels during convalescence would enable repeat testing if a new or unresolved infection is suspected, in comparison with standard ELISA and Western blot tests, which can remain positive for decades after infection (31). Here, anti-PA and anti-PC antibodies were found to reach approximately 85% of their maximum titer on the day of diagnosis, highlighting their potential application for diagnosis early in the course of infection. This may partly be due to the distinct processing of lipid antigens but may also be a function of their status as autoantibodies. Stimuli driving derepression of existing autoantibodies, already circulating at low levels, may increase titers more rapidly than occurs with antibodies generated de novo by uniquely bacterial antigens.

Previous screens for antibodies raised during *Borrelia* infection have been genomic (64, 65), proteomic (66, 67), or peptide based (68) and therefore neglect the lipid component of cells. The identification of IgG antiphospholipids - in addition to the known antigalactosylcholesterol (14) - expands the repertoire of antibodies that may have diagnostic potential. Further investigation of the antibodies described here (and potentially other anti-lipids) may reveal them to be valuable diagnostic tools. The prevalence of these antiphospholipid antibodies in the healthy population and in other infectious and inflammatory diseases will need to be determined to understand the specificity of the responses identified here. Given the presence of antiphospholipid antibodies in numerous infectious and inflammatory diseases, it is possible that the responses may lack the specificity required for broad use as a standalone test for Lyme disease. Nevertheless, antiphospholipid antibody testing could be a valuable adjunctive test to standard antibody testing, one that is useful for identifying early infection, repeat infection, and response to therapy.

As described herein, the STT test is currently the only format of test recommended by the CDC for *B. burgdorferi* testing, with the majority of the tests in the market based upon this format. These tests differ from the method of the instant application as they use antibodies to a series of proteins, rather than lipids. The STT test is a two-stage test and uses a panel of 15 antibodies, split into two groups. Different combinations are used depending on the length of the patient's symptoms. The first tier is sensitive but not specific: a positive result in ELISA #1 reflexes the patient to the second tier, which is more specific. Exactly how these tests are performed varies from test to test; however, the antibodies used in the tests are the same. While traditional versions are called the STT (standardized two-tier test), more modern versions of the test are referred to as a MTT (modified two-tier test) which use another set of ELISAs instead of the Western blots in the second tier. These tests generally look for the same antibodies.

The STT and MTT have several limitations, as demonstrated by the comparison of the STT with the anti-lipid test of the current disclosure (Figure 1). The STT is not sensitive when testing in the first 2-3 weeks of infection, as shown in figures 4 and 5, and they are complex to run and interpret. For these reasons and others, the CDC states that the STT/MTT should not be be used to track patients during treatment and recovery. When compared to the αP lipid-detection test of the current disclosure, the αP is capable of detection of infection within one week (Figure. 6) and is considerably less complex than the STT or MTT (Figure 7).
In embodiments, one or more steps of the anti-lipid method is integrated into the STT or MTT. For example, the MTT may be modified to include testing for one or more anti-lipid antibodies (e.g., within the first tier or second tier of the exiting STT/MTT panel). In another example, the anti-lipid method may be used to track patients after treatment. For example a patient may have an initial diagnosis using the STT/MTT tests, then the anti-lipid test is used to track the patient after treatment.

### Methods

### Culture conditions and delipidated growth medium.

*B. burgdorferi* B31 was routinely cultured in BSK medium composed of BSA (50.00 g/L), CMRL-1066 (9.80, US Biologicals), HEPES (6.60), peptone (5.60), dextrose (5.60), sodium bicarbonate (2.44), yeast extract (2.20), sodium pyruvate (1.00), sodium citrate (0.90), *N-*acetyl glucosamine (0.50), and 6.2% rabbit serum. Media were filter-sterilized and the pH adjusted to 7.6 before the addition of gelatin to 1.4% and sterile water to 1 L, followed by storage at -20°C. The *B. burgdorferi* B31 isolate used throughout this study was an infectious strain, cultured from a mouse. By PCR plasmid typing, this strain possessed all B31 plasmids except lp5, cp32-6, and cp32-9, which are not known to carry genes related to either virulence or metabolism.

dBSK was prepared as described above except for the omission of gelatin, which is not essential (36). Defined components were combined as a 5× stock and stored at -20°C. BSA, serum, and yeast extract were mixed to 2× in 50 mL, to which an equal volume of 2:1 chloroform/methanol was added and stirred for 90 minutes. Organic and aqueous phases were separated by centrifugation at 800g for 10 minutes. The aqueous phase was retained and underwent 2 further identical extractions. Residual methanol was evaporated overnight under a flow of air, and the 2× delipidated mixture was made to complete medium by addition of the defined stock solution and water to 100 mL, followed by pH adjustment to 7.6 and filter sterilization. The dBSK was stored at -20°C.

For growth curves, fatty acids and cholesterol (Avanti) were stored in chloroform at 50 mM. Lipids at the requisite concentrations were added to 1.5 mL microfuge tubes and the chloroform evaporated under vacuum before resuspension in culture medium. *B. burgdorferi* was added to a density of 1 × 10⁵ from a stationary phase culture and incubated at 32°C. Cells were directly enumerated under darkfield microscopy using a Petroff-Hausser counter (Hausser Scientific) daily for 10 days and diluted in PBS at later time points.

### Incorporation of fluorescent lipid analogs.

Cells were grown to a stationary phase (5 days) in BSK and then pelleted (5 minutes at 3220g), washed once in an equal volume of PBS, and resuspended in an equal volume of dBSK. The cell suspension (1 mL) was added to nitrobenzoxadiazole-labeled (NBD-labeled) phospholipids (Avanti) to 25 µM. At hourly intervals, the cells were pelleted (5 minutes at 18,800g), washed 3 times in 1 mL PBS, and resuspended in 100 µL PBS. The suspensions were transferred onto clear-bottomed, opaque 96-well plates (Corning), and fluorescence was recorded at excitation 460 nm/emission 540 nm (Ex₄₆₀/Em₅₄₀). *Staphylococcus aureus* 502a and *Escherichia coli* MG1655 were grown overnight in tryptic soy broth (TSB): tryptone (17.0 g/L), soytone (3.0), glucose (2.5), sodium chloride (5.0), and dipotassium phosphate (2.5). Incorporation of NBD-phospholipids was carried out in TSB, with subsequent washing and measurement as above.

After fluorescence measurements, 5 µL cell suspensions were spotted onto glass slides and fixed with 5 µL ProLong Gold (Thermo Fisher Scientific) with DAPI. The slides were imaged using a Leica SP8 Falcon with line-sequential imaging of DAPI (Ex = 405 nm, detector = 410-466 nm) and NBD (excitation = 470 nm, detector 550-600 nm) to avoid crosstalk. After acquisition, the images were enhanced using the Leica LAS X software package. Images brightness and contrast were enhanced to illustrate both the fluorescent dye and DAPI staining, with untreated controls processed in the same manner as the experimental images. For all 3 bacterial species imaged, the laser power was calibrated such that minimal autofluorescence was detected in unlabeled cells prior to imaging of the labeled cells.

### Murine infection and endpoint ELISA.

Six female 8-week-old C3H-HeJ mice (The Jackson Laboratory) were infected s.c. with 1 × 10⁴ *B. burgdorferi* B31 in 100 µL sterile PBS. Infection was confirmed by culturing of *B. burgdorferi* from an ear-punch biopsy taken on day 14. Serum was collected after euthanasia on day 28. Serum was collected after centrifugation for 2 minutes at 18,800g and stored at -20°C. Serial samples were collected from 3 male and 3 female 8-week-old mice (The Jackson Laboratory) infected as above, with blood drawn weekly by submandibular punch. Serum was collected from blood as described above.

Hydrophobic Immulon-B-coated, flat-bottomed 96-well plates (Thermo Fisher Scientific) were coated with 1 µg lipid in 100 µL ethanol, which was evaporated overnight. The coated plates were washed twice with 200 µL PBS and blocked for 3 hours at room temperature with 150 µL 1% BSA in PBS, which was then removed by 3 further washes with PBS. Sera were stored diluted in PBS and then serially diluted in 2-fold steps. Sera were incubated at room temperature for 2.5 hours, along with 6 or 12 PBS-only controls per plate. After 3 washes (aspirated by pipetting) with 200 µL PBS plus 0.1% Tween-80, 100 µL goat anti-mouse IgG (H+L, Thermo Fisher Scientific, A16066) at 1:2000 in PBS was added to each well and incubated for 1 hour. Plates were washed 3 times with PBS plus 0.1% Tween-80. One hundred microliters TMB peroxidase substrate (KPL SureBlue, SeraCare) per well was incubated for 5-10 minutes until color developed, and the reaction was stopped with 100 µL 1N HCl. OD₄₅₀ was recorded in a Biotek Synergy HT plate reader; the endpoint titer was the highest dilution at which the average OD₄₅₀ of 3 technical replicates remained higher than the greatest of 12 serum-free controls plus their SD.

### Patient samples.

Discarded human sera from 24 patients undergoing evaluation for Lyme disease were collected, and their medical records were reviewed. The duration of infection was determined from the self-reported date of onset of symptoms (1-75 days prior to sample collection). Untreated patients had the diagnosis of Lyme disease established by the presence of erythema migrans (documented by a clinician) or by facial palsy or arthritis confirmed by a positive STT test. For the untreated patient group, samples were collected prior to antibiotic treatment. Patients in the post-treatment group were all at least 1 year from the onset of infection and initial treatment. Although some individuals had continuing, nonspecific symptoms, none in the post-treatment group had evidence of a new infection: those with a new EM or tick exposure were excluded. Control sera were either collected from healthy volunteers or purchased from SeraCare, which collected the sera from healthy individuals residing in Florida, where Lyme disease is not endemic. Syphilis sera were purchased from Precision Biomedicine, which sourced the sera from patients positive by both treponemal (syphilis IgG) and nontreponemal (RPR) tests.

### Single-dilution ELISA

For single-dilution ELISAs, Immulon-B-coated, round-bottomed 96-well plates (Thermo Fisher Scientific) were coated, washed, and blocked as described above. Sera were diluted to 1:25,000 in PBS and incubated on plates (in triplicate) for 2.5 hours. Five washes with 200 µL PBS plus 0.1% Tween-80 were performed by inversion of the plate, after which 100 µL goat anti-human IgG (H+L, Thermo Fisher Scientific, A18805) at 1:2000 (0.5 µg/mL) in PBS was added. Plates were washed 5 times in 200 µL PBS plus 0.1% Tween-80 and developed with 100 µL TMB peroxidase substrate for 5 minutes exactly, after which the reactions were stopped with 100 µL 1N HCl and read as above. All values were normalized to the average of at least 6 naive controls run on the same plate to yield an anti-PL index, where an index of greater than 1 indicated a titer above the mean of the controls, and less than 1 indicated a titer below this mean. Three technical replicates for each sample were averaged, and the data presented are the combined average of data from 3 independent plates. The cutoff value for positivity was the mean of the naive control group plus 2.291 SDs. This was determined using the statistical method of Frey et al. (35) for a group of 12 samples and a 97.5% CI.

### Statistics

Simple linear regression analyses (for correlations between single-dilution antibody titers and titer versus collection date), Tukey's tests (for multiple comparisons), and unpaired, 2-tailed t tests (for endpoint and single-dilution ELISAs) were performed using GraphPad Prism, version 9 (GraphPad Software).

### Study approval

The animal experiments were conducted under the supervision of the IACUC of Tufts University, an institution approved by the Office of Laboratory Animal Welfare at the NIH and accredited by the Association for Assessment and Accreditation of Laboratory Animal Care (International) (AAALAC). Serial serum samples from patients with single or multiple erythema migrans lesions were obtained under a clinical protocol (ClinicalTrials.gov identifier NCT00028080). The study was approved by the IRB of the NIAID, NIH. Written informed consent was obtained from all participants. All patients fulfilled the US Centers for Disease Control and Prevention 2017 Lyme Disease case definition criteria.

### Example 2: Lipid antibodies as a diagnostic test for Lyme disease

Antibody titers in persistent Lyme disease. A percentage of people who get Lyme disease do not recover after treatment. The anti-lipid antibody test may be used to predict or diagnose patients with persistent and/or chronic Lyme disease symptoms.

Figure 18 shows two groups of patients: those who recovered and had no symptoms after treatment (R) and those whose symptoms persisted (P). The persistent symptoms group largely have higher antibody titers, suggesting that the test could be useful. We expect that patients with chronic symptoms will be a heterogenous group, with disease from several different causes.

### Specificity

Panels of samples from patients were tested with various other conditions that might interfere with diagnosis (Figure 19) These are either diseases with similar clinical presentation (autoimmune conditions: lupus, rheumatoid arthritis, multiple sclerosis) or known to raise similar anti-lipid antibodies (syphilis, HIV). We also tested another common tickborne disease, babesia. In some cases, relatively small differences were seen between healthy controls (C) and the various disease sera. Therefore, care must be taken when determining cutoff values. Larger sample sizes may also help to maximize true positives and minimize false negatives.

On way to perform these statistics is to use receiver operating characteristic (ROC) curves, which graph the number of true- and false- positives for different cutoffs. In short, the closer the curve is to the top left of the graph, the greater the expectation of true positives. A 50% successful diagnosis (e.g., tossing a coin to decide) would have a straight line through the center of the graph. Diagnostic efficiency is quantified by measuring the area under the curve. Figure 20 demonstrates ROC curves for distinguishing Lyme infections from healthy or diseased controls (i.e., using the test to diagnose acute infections) and figure 21 shows curves for distinguishing post-treatment individuals from active Lyme infections (i.e. using the test to track treatment). In this instance, the test appears to be better at tracking treatment (total area under the curve: 2.78) than detecting acute infections (2.51).

### Additional Antigens

Other types and families of lipid antibodies may be used for diagnosis of Lyme disease and detection of *Boriella sp.* infection in humans and other animals. For example, antibodies used for detection may include antibodies for phospholipids PG, PC, PA, PS, PE, and the PG dimer, CL, as disclosed herein. Other antigens which would generate antibodies useful for detection and/or diagnosis of *Boriella sp.* infection may include sphingolipids such as ceramide and sphingomyelin, as shown in figures 5A and 5B, respectively. Other antigens may also include galactosylcholesterol esters and galactosyldiacylglycerol.

### Example 3: Lipids found in Borrelia membranes.

In some embodiments, the methods include testing for one or more lipids, or antibodies generated against one or more lipids found in *Boriella sp.,* such as *B. burgdorferi.* It has been previously shown that *B. burgdorferi* takes up lipids from their environments (69, 70), and that some of the bacterium's own lipids are antibody targets (70, 71). Therefore, it is likely that many, if not all, of the lipids found on the *Borrelia* membranes are candidates for detection of antibodies against these lipids in infected subjects. Figures 23-24 are graphs illustrating the detection of membrane lipids in *B. burgdorferi* under differing media conditions. These results are combined in Table 7 to show a list of *Borrelia* membranes lipids identified by the inventors, and which could be used in a panel to detect *Borrelia. sp.* infection.

**Table 7**

| **(Lyso-) phospholipids** | **Sterols and sterol derivatives** | **Sphingolipids** | **Diacylglycerols** |
|---|---|---|---|
| Phosphatidylinositol (PI) | Hexosyl campesterol | Hexosylceramide | Diacylglycerol |
| Phosphatidylinositol phosphate (PIP2) | Hexosyl campesterol esters | Phosphoceramide | Monoacylglycerol |
| Lyso PC | Campesterol | | Hexosyl monoacylglycerol |
| Lyso PE | Campesterol esters | | Hexosyl diacylglycerol |
| Lyso PG | Hexosyl sitosterol | | |
| Bis-methyl PA | Hexosyl sitosterol esters | | |
| Cardiolipin | Sitosterol | | |
| Dimethyl PE | Sitosterol esters | | |
| Methyl PC | Hexosyl stigmasterol | | |
| | Hexosyl stigmasterol esters | | |
| | Hexosyl Zymesterol | | |
| | Hexosyl Zymesterol esters | | |
| | Zymosterol | | |
| | Zymosterol esters | | |

### Example 4: Taxonomy studies of the genus Borreliaceae

Figure 25 is a flowchart illustrating the taxonomy of *Borreliaceae* species. Not all *Borreliaceae* species are listed. The members of the genus *Borreliaceae* are genetically and biologically similar, and it is likely that the lipid biology between species is similar enough that antibodies identified for one *Borreliaceae* species may also be detected for another *Borreliaceae* species. The rationale that the core biology of lipid metabolism is shared across *Borreliaceae* species is three-fold. First, all members of the genus *Borreliaceae* have similar (small genomes). For example, members of the genus have a chromosome of between 900-1000 kilobases and a selection of plasmids that are closely related between the various species. Small genomes have limited metabolisms (e.g., limited by the small number of genes encoded within the small genome) so all members of the species are likely to be limited in a similar fashion. For this reason, it is likely that most or all *Borreliaceae* species are dependent on lipids scavenged from the environment.

Second, lipids can incorporate into *Borreliaceae* species via diffusion, a mechanism that is enabled at least partially by the absence of a protective liposaccharide capsule in these bacteria (e.g., the absence of a capsule in *Borreliaceae,* as well as other capsule-less bacteria, such as Treponema, making the membrane more accessible so that lipids may drift in). Once incorporated into the bacterial membrane, the lipids may then be targeted for immune detection and the production of anti-lipid antibodies. Therefore, *Borreliaceae* species may accumulate lipids in the same manner, and may possibly generate the same or similar antibodies.

Third, the similarity of biological processes between *Borreliaceae* species is well known. For example, a paper by Livermore et al. studied *B. hermsii* and showed some similar basic biology to that which was later described in *B. burgdorferi,* Namely, that the typical composition of the membranes are similar, and that both incorporate fatty acids and cholesterol from their environment (73). Neither species is capable of synthesizing fatty acids *de novo.* Like *B. burgdorferi,* the fatty acid composition of *B. hermsii* reflects that of the growth medium. These results suggest that the anti-lipid test of this disclosure may be used for the detection of other *Borreliaceae* species or the diagnosis of infection by other *Borreliaceae* species. For example, the method may be used for the detection of *B. mayonii,* the second main *Borreliaceae* species causing cases of Lyme disease in the United States. In another example, the method may be used for diagnosis and/or detection of one or more *Borreliaceae* species causing European Lyme disease (e.g., *B. afzelii* and *B. garinii*)*.* In another example, the method may be used for diagnosis and/or detection of one or more *Borreliaceae* species causing tick-borne relapsing fever (e.g., *B. hermsii, B. recurrentis, B. turicatae,* and *B. miyamotoi*)*.*

### References

1. Schutzer SE, et al. Whole-genome sequences of thirteen isolates of Borrelia burgdorferi. J Bacteri- ol. 2011;193(4):1018-1020.
2. Moran NA. Microbial minimalism: genome reduction in bacterial pathogens. Cell. 2002;108(5):583-586.
3. Bontemps-Gallo S, et al. Borrelia burgdorferi genes, bb0639-0642, encode a putative putres- cine/spermidine transport system, PotABCD, that is spermidine specific and essential for cell survival. Mol Microbiol. 2018;108(4):350-360.
4. Wyss C, Ermert P. Borrelia burgdorferi is an adenine and spermidine auxotroph. Microb Ecol Health Dis. 1996;9(4):181-185.
5. Schneider EM, Rhodes RG. N-acetylmannosamine (ManNAc) supports the growth of Borrelia burgdorferi in the absence of N-acetylglucosamine (GlcNAc). FEMS Microbiol Lett. 2018;365(21):fny243.
6. Ben-Menachem G, et al. A newly discovered cho- lesteryl galactoside from Borrelia burgdorferi. Proc Natl Acad Sci USA. 2003;100(13):7913-7918.
7. Wang XG, et al. Phospholipid synthesis in Borrelia burgdorferi: BB0249 and BB0721 encode functional phosphatidylcholine synthase and phos-phatidylglycerolphosphate synthase proteins. Microbiology (Reading). 2004;150(2):391-397.
8. Östberg Y, et al. Functional analysis of a lipid galactosyltransferase synthesizing the major envelope lipid in the Lyme disease spirochete Borrelia burgdorferi. FEMS Microbiol Lett. 2007;272(1):22-29.
9. Hossain H, et al. Structural analysis of glycolipids from Borrelia burgdorferi. Biochimie. 2001;83(7):683-692.
10. Radolf JD, et al. Characterization of outer membranes isolated from Borrelia burgdorferi, the Lyme disease spirochete. Infect Immun. 1995;63(6):2154-2163.
11. Livermore BP, et al. Lipid metabolism of Borrelia hermsii. Infect Immun. 1978;20(1):215-220.
12. Befisle JT, et al. Fatty acids of Treponema pallidum and Borrelia burgdorferi lipoproteins. J Bacteriol. 1994;176(8):2151-2157.
13. Crowley JT, et al. Lipid exchange between Borrelia burgdorferi and host cells. PLoS Pathog. 2013;9(1):e1003109.
14. Jones KL, et al. Strong IgG antibody responses to Borrelia burgdorferi glycolipids in patients with Lyme arthritis, a late manifestation of the infection. Clin Immunol. 2009;132(1):93-102.
15. Garcia-Monco JC, et al. Experimental immunization with Borrelia burgdorferi induces develop- ment of antibodies to gangliosides. Infect Immun. 1995;63(10):4130-4137.
16. Tatituri RVV, et al. Recognition of microbial and mammalian phospholipid antigens by NKT cells with diverse TCRs. Proc Natl Acad Sci USA. 2013;110(5): 1827-1832.
17. Olson CM, et al. Local production of IFN-gamma by invariant NKT cells modulates acute Lyme carditis. J Immunol. 2009;182(6):3728-3734.
18. Lee WY, et al. An intravascular immune response to Borrelia burgdorferi involves Kupffer cells and i NKT cells. Nat Immunol. 2010;11(4):295-302.
19. Goodridge A, et al. Anti-phospholipid antibody levels as biomarker for monitoring tuberculosis treatment response. Tuberculosis (Edinb). 2012;92(3):243-247.
20. Ordi-Ros J, et al. Prevalence, significance, and specificity of antibodies to phospholipids in Q fever. Clin Infect Dis. 1994;18(2):213-218.
21. Barber BE, et al. Antiphosphatidylserine immunoglobulin m and immunoglobulin g antibodies are higher in vivax than falciparum malaria, and associated with early anemia in both species. J Infect Dis. 2019;220(9):1435-1443.
22. Arbuckle MR, et al. Development of autoantibodies before the clinical onset of systemic lupus erythematosus. N Engl J Med. 2003;349(16):1526-1533.
23. Cervera R et al. Antiphospholipid syndrome: clin- ical and immunologic manifestations and pat- terns of disease expression in a cohort of 1,000 patients. Arthritis Rheum. 2002;46(4):1019-1027.
24. Janoff AS, Rauch J. The structural specificity of anti-phospholipid antibodies in autoimmune disease. Chem Phys Lipids. 1986;40(2):315-332.
25. Matthews HM, et al. Unique lipid composition of Treponema pallidum (Nichols virulent strain). Infect Immun. 1979;24(3):713-719.
26. Gao K, et al. Origin of nontreponemal anti- bodies during Treponema pallidum infection: evidence from a rabbit model. J Infect Dis. 2018;218(5):835-843.
27. Henao-Martínez AF, Johnson SC. Diagnostic tests for syphilis: new tests and new algorithms. Neurol Clin Pract. 2014;4(2):114-122.
28. Marques A, et al. The widening gyre: controversies in lyme disease. In: Radolf, JD, Samuels DS, eds. Lyme Disease and Relapsing Fever Spirochetes: Genomics, Molecular Biology, Host Interactions and Disease Pathogenesis. Caister Academic Press; 2021:685-702
29. Marques AR. Revisiting the lyme disease serodiagnostic algorithm: the momentum gathers. J Clin Microbiol. 2018;56(8):e00749-18.
30. Mead P. Updated CDC recommendation for serologic diagnosis of lyme disease. MMWR Morb Mortal Wkly Rep. 2019;68(32):703.
31. Pegalajar-Jurado A, et al. Evaluation of modified two-tiered testing algorithms for lyme disease laboratory diagnosis using well-characterized serum samples. J Clin Microbiol. 2018;56(8):e01943-17.
32. Kalish RA, et al. Persistence of immunoglobulin M or immunoglobulin G antibody responses to Borrelia burgdorferi 10-20 years after active lyme disease. Clin Infect Dis. 2001;33(6):780-785.
33. Stübs G, et al. Acylated cholesteryl galactosides are specific antigens of Borrelia causing Lyme disease and frequently induce anti- bodies in late stages of disease. J Biol Chem. 2009;284(20):13326-13334.
34. Ratnam S. The laboratory diagnosis of syphilis. Can J Infect Dis Med Microbiol. 2005;16(1):45-51.
35. Frey A, et al. A statistically defined endpoint titer determination method for immunoassays. J Immunol Methods. 1998;221(1):35-41.
36. Pollack RJ, et al. Standardization of medium for culturing Lyme disease spirochetes. J Clin Micro- biol. 1993;31(5):1251-1255.
37. Barbour AG. Isolation and cultivation of Lyme disease spirochetes. Yale J Biol Med. 1984;57(4):521-525.
38. Lackum K von, Stevenson B. Carbohydrate utilization by the Lyme borreliosis spirochete, Borrelia burgdorferi. FEMS Microbiol Lett. 2005;243(1):173-179.
39. Posey JE, Gherardini FC. Lack of a role for iron in the Lyme disease pathogen. Science. 2000;288(5471):1651-1653.
40. Brinster S, et al. Type II fatty acid synthesis is not a suitable antibiotic target for Gram-positive pathogens. Nature. 2009;458(7234):83-86.
41. Brennan PJ, et al. Invariant natural killer T cells recognize lipid self antigen induced by microbial dan- ger signals. Nat Immunol. 2011;12(12):1202-1211.
42. Kinjo Y, et al. Invariant natural killer T cells recognize glycolipids from pathogenic Gram-posi- tive bacteria. Nat Immunol. 2011;12(10):966-974.
43. Wolf BJ, et al. Identification of a potent microbial lipid antigen for diverse natural killer T cells. J Immunol. 2015;195(6):2540-2551.
44. Cox DL, Radolf JD. Insertion of fluorescent fatty acid probes into the outer membranes of the pathogenic spirochaetes Treponema pallidum and Borrelia burgdorferi. Microbiology (Reading). 2001;147(5):1161-1169.
45. Rego ROM, et al. Population bottlenecks during the infectious cycle of the Lyme disease spirochete Borrelia burgdorferi. PLoS One. 2014;9(6):e101009.
46. Wallach FR, et al. Circulating Borrelia burgdorferi in patients with acute Lyme disease: results of blood cultures and serum DNA analysis. J Infect Dis. 1993;168(6): 1541-1543.
47. Quehenberger O, et al. Lipidomics reveals a remarkable diversity of lipids in human plasma. J Lipid Res. 2010;51(11):3299-3305.
48. Jain M, et al. A systematic survey of lipids across mouse tissues. Am J Physiol Endocrinol Metab. 2014;306(8):E854-E868.
49. Pradas I, et al. Lipidomics reveals a tissue-specific fingerprint. Front Physiol. 2018;9:1165.
50. Kent C, et al. A CDP-choline pathway for phosphatidylcholine biosynthesis in Treponema denti- cola. Mol Microbiol. 2004;51(2):471-481.
51. Mackworth-Young CG, et al. Anticardiolipin antibodies in Lyme disease. Arthritis Rheum. 1988;31(8):1052-1056.
52. Moncó JCG, et al. Reactivity of neuroborreliosis patients (Lyme disease) to cardiolipin and gangli- osides. J Neurol Sci. 1993;117(1):206-214.
53. Soreng K, et al. Serologic testing for syphilis: benefits and challenges of a reverse algorithm. Clin Microbiol Newsl. 2014;36(24):195-202.
54. Kinjo Y, et al. Natural killer T cells recognize diacylglycerol antigens from pathogenic bacteria. Nat Immunol. 2006;7(9):978-986.
55. Beermann C, et al. The lipid component of lipoproteins from Borrelia burgdorferi: structural analysis, antigenicity, and presentation via human dendritic cells. Biochem Biophys Res Com- mun. 2000;267(3):897-905.
56. de los Angeles Garcia M, et al. Evaluation of specific humoral immune response and cross reac- tivity against Mycobacterium tuberculosis antigens induced in mice immunized with liposomes composed of total lipids extracted from Mycobacterium smegmatis. BMC Immunol. 2013;14(1):S11.
57. Grazioli S, Pugin J. Mitochondrial damage-associated molecular patterns: from inflammatory signaling to human diseases. Front Immunol. 2018;9:832.
58. Lehane A, et al. Prevalence of single and coinfections of human pathogens in Ixodes ticks from five geographical regions in the United States, 2013-2019. Ticks Tick-borne Dis. 2021;12(2):101637.
59. Waddell LA, et al. The accuracy of diagnostic tests for Lyme disease in humans, a systematic review and meta-analysis of North American Research. PLoS One. 2016;11(12):e0168613.
60. Chang PP, et al. Identification of Bcl-6-depen- dent follicular helper NKT cells that provide cognate help for B cell responses. Nat Immunol. 2012;13(1):35-43.
61. Doherty DG, et al. Activation and regulation of B cell responses by invariant natural killer T cells. Front Immunol. 2018;9:1360.
62. King IL, et al. Invariant natural killer T cells direct B cell responses to cognate lipid antigen in an IL-21-dependent manner. Nat Immunol. 2012;13(1):44-50.
63. Sar1c1 SU, et al. Anticardiolipin antibodies in children with Helicobacter pylori infection. Helicobacter. 2015;20(6):418-421.
64. Mueller M, et al. Identification of Borrelia burgdorferi ribosomal protein L25 by the phage surface display method and evaluation of the protein's value for serodiagnosis. J Clin Microbiol. 2006;44(10):3778-3780.
65. Barbour AG, et al. A genome-wide proteome array reveals a limited set of immunogens in natural infections of humans and white-footed mice with Borrelia burgdorferi. Infect Immun. 2008;76(8):3374-3389.
66. Batool M, et al. Identification of surface epi- topes associated with protection against highly immune-evasive VlsE-expressing lyme disease spirochetes. Infect Immun. 2018;86(8):e00182-18.
67. Harris EK et al. Immunoproteomic analysis of Borrelia miyamotoi for the identification of sero- diagnostic antigens. Sci Rep. 2019;9(1):16808.
68. Ionov Y, Rogovskyy AS. Comparison of motif- based and whole-unique-sequence-based analyses of phage display library datasets generated by biopanning of anti-Borrelia burgdorferi immune sera. PLoS One. 2020;15(1):e0226378.
69. Crowley JT, Toledo AM, LaRocca TJ, Coleman JL, London E, et al. (2013) Lipid Exchange between Borrelia burgdorferi and Host Cells. PLOS Pathogens 9(1): e1003109.
70. Cox DL, Radolf JD. Insertion of fluorescent fatty acid probes into the outer membranes of the pathogenic spirochaetes Treponema pallidum and Borrelia burgdorferi. Microbiology (Reading). 2001 May;147(Pt 5):1161-1169. doi: 10.1099/00221287-147-5-1161. PMID: 11320119.
71. Schröder NW, Schombel U, Heine H, Göbel UB, Zähringer U, Schumann RR. Acylated cholesteryl galactoside as a novel immunogenic motif in Borrelia burgdorferi sensustricto. J Biol Chem. 2003 Sep 5;278(36):33645-53. doi: 10.1074/jbc.M305799200. Epub 2003 Jun 16. PMID: 12810705.
72. Jones KL, Seward RJ, Ben-Menachem G, Glickstein LJ, Costello CE, Steere AC. Strong IgG antibody responses to Borrelia burgdorferi glycolipids in patients with Lyme arthritis, a late manifestation of the infection. Clin Immunol. 2009 Jul;132(1):93-102. doi: 10. 10 1 6/j.clim.2009.03.5 10. Epub 2009 Apr 2. PMID: 19342303; PMCID: PMC2752957.
73. Livermore BP, Bey RF, Johnson RC. Lipid metabolism of Borrelia hermsi. Infect Immun. 1978 Apr;20(1):215-20. doi: 10.1128/iai.20.1.215-220.1978. PMID: 669794; PMCID: PMC421574.

All methods described herein can be performed in any suitable order unless otherwise indicated herein or otherwise clearly contradicted by context. The use of any and all examples provided herein, is intended merely to better illuminate the invention and does not pose a limitation on the scope of the invention unless otherwise claimed. No language in the specification should be construed as indicating any non-claimed element as essential to the practice of the invention.

Citations to a number of patent and non-patent references are made herein. The cited references are incorporated by reference herein in their entireties. In the event that there is an inconsistency between a definition of a term in the specification as compared to a definition of the term in a cited reference, the term should be interpreted based on the definition in the specification.

In the foregoing description, it will be readily apparent to one skilled in the art that varying substitutions and modifications may be made to the invention disclosed herein without departing from the scope and spirit of the invention. The invention illustratively described herein suitably may be practiced in the absence of any element or elements, limitation or limitations which is not specifically disclosed herein. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention. Thus, it should be understood that although the present invention has been illustrated by specific embodiments and optional features, modification and/or variation of the concepts herein disclosed may be resorted to by those skilled in the art, and that such modifications and variations are considered to be within the scope of this invention.

## Claims

1. A method comprising:
(a) contacting an antibody-containing sample, derived from a subject, with a lipid panel configured on a solid support, the lipid panel comprising at least the following lipids: phosphatidic acid (PA), phosphatidyl choline (PC), and phosphatidylserine (PS), and optionally comprising one or more of ceramide, sphingomyelin, hexosyl cholesterol, acyl hexosyl cholesterol, galactosyldiacylglycerol, and phosphatidylethanolamine;
(b) incubating the sample with the lipids to allow binding of antibodies in the sample to the lipids;
(c) contacting the bound antibodies with a detectable binding agent;
(d) detecting the detectable binding agent;
(e) measuring the level of antibodies bound to each of the lipids based on (d);
(f) comparing the measured levels of (e) to a predetermined value.

2. The method of claim 1, wherein the sample comprises a blood sample, a plasma sample, or a serum sample.

3. The method of claim 1, wherein the solid support comprises a bead, plate, test strip, or flow cell.

4. The method of claim 1, wherein the subject is human and the detectable binding agent comprises anti-human IgG comprising a detectable label.

5. The method of any one of claims 1-4, wherein the subject has been exposed to a Lyme disease pathogen for one week or less.

6. The method of any one of claims 1-4, wherein if the measured level is greater than the predetermined value for at least one of the lipids in the panel, the subject is diagnosed as positive for Lyme disease.

7. The method of claim 6, further comprising treating the subject for Lyme disease.

8. A method comprising:
(a) contacting an antibody-containing sample, derived from a subject, with a reagent composition comprising the following lipids: phosphatidic acid (PA), phosphatidyl choline (PC), and phosphatidylserine (PS); the reagent composition optionally including one or more additional lipids selected from ceramide, sphingomyelin, hexosyl cholesterol, acyl hexosyl cholesterol, galactosyldiacylglycerol, and phosphatidylethanolamine;
(b) incubating the sample with the reagent composition to allow binding of antibodies in the sample, if present, to the lipids thereby forming an antibody-lipid complex;
(c) detecting bound antibodies or antibody-lipid complexes;
(d) measuring the level of antibodies bound to PA, PC, and PS, and optionally the level of the one or more additional lipids based on (c); and
(e) comparing the measured levels of (d) to a predetermined value for PA, PC, and PS and any of the additional lipids measured.

9. The method of claim 8, wherein the subject has been exposed to a Lyme disease pathogen for one week or less.

10. The method of any one of claims 8-9, wherein if the measured level is greater than the predetermined value for at least one of the lipids in the panel, the subject is diagnosed as positive for Lyme disease.

11. The method of claim 10, further comprising treating the subject for Lyme disease.

12. A composition comprising:
phosphatidic acid (PA), phosphatidyl choline (PC), phosphatidylserine (PS); and at least one of a synthetic buffer, stabilizer, non-specific binding surface blocking agent, macromolecular crowding agent, antioxidant, preservative, and surfactant, wherein the PA, PC, and PS, are immobilized on a solid support, wherein the solid support comprises a plate, bead, flow chamber, microfluidic chamber, or microchip comprising a plurality of microfluidic chambers.

13. The composition of claim 12, comprised within a kit for detection of infection with *Borrelia sp.*

14. The composition of claim 13, wherein the *Borrelia* species is one of *Borrelia afzelii, Borrelia burgdorferi, Borrelia garinii,* or *Borrelia mayonii.*

15. The composition of any one of claims 12-14, further comprising at least one of ceramide, sphingomyelin, hexosyl cholesterol, acyl hexosyl cholesterol, galactosyldiacylglycerol, or phosphatidylethanolamine, wherein the ceramide, sphingomyelin, hexosyl cholesterol, acyl hexosyl cholesterol, galactosyldiacylglycerol, or phosphatidylethanolamine immobilized on the solid support.
